(19) Europäisches Patentamt
European Patent Office
Office européen des brevets

(11) Publication number: **0 619 298 A2**

# (12) EUROPEAN PATENT APPLICATION

(21) Application number: **94105516.2**

(22) Date of filing: **11.04.94**

(51) Int. Cl.$^5$: **C07C 251/58**, C07D 295/12, A61K 31/15, A61K 31/445, A61K 31/495

(30) Priority: **09.04.93 HU 104193**

(43) Date of publication of application: **12.10.94 Bulletin 94/41**

(84) Designated Contracting States: **AT BE CH DE DK ES FR GR IT LI NL SE**

(71) Applicant: **EGIS GYOGYSZERGYAR RT.**
**Kereszturi ut 30-38**
**H-1106 Budapest (HU)**

(72) Inventor: **Bajnogel, Judit Dr.**
**Benkö I. u. 25**
**H-1151 Budapest (HU)**
Inventor: **Blasko, Gábor Dr.**
**Molinár E. u. 21**
**H-1113 Budapest (HU)**
Inventor: **Budai, Zoltán Dr.**
**Lukács u. 3**
**H-1023 Budapest (HU)**
Inventor: **Egyed, András Dr.**
**Ujvidék u. 58**
**H-1145 Budapest (HU)**
Inventor: **Fekete, Márton Dr.**
**Fö u. 49**
**H-1027 Budapest (HU)**
Inventor: **Karaffa, Erika chemist**
**Pesti u. 58**
**H-1173 Budapest (HU)**
Inventor: **Mezei, Tibor Dr.**
**Borz u. 4**
**H-1221 Budapest (HU)**
Inventor: **Reiter, Klára Dr.**
**Tövis u. 32/b**
**H-1022 Budapest (HU)**
Inventor: **Simig, Gyula Dr.**
**Hollosy S. u. 25**
**H-1126 Budapest (HU)**
Inventor: **Szemerédi, Katalin Dr.**
**Vörösvári u. 3**
**H-1035 Budapest (HU)**
Inventor: **Szirt, Enikö biologist**
**Téglavetö u. 24**
**H-1105 Budapest (HU)**

(74) Representative: **Beszédes, Stephan G., Dr.**
**Patentanwalt**
**Postfach 11 68**
**D-85201 Dachau (DE)**

(54) **(Phenylmethylene)-2-(alkyl)-1- omega-(amino)-alkoxyimino -cycloalkane derivatives, a process for preparing them as well as medicaments containing them and the use of them.**

(57) A subject-matter of the invention are (phenylmethylene)-2-(alkyl)-1-[ω-(amino)-alkoxyimino]-cycloalkane derivatives of general formula

EP 0 619 298 A2

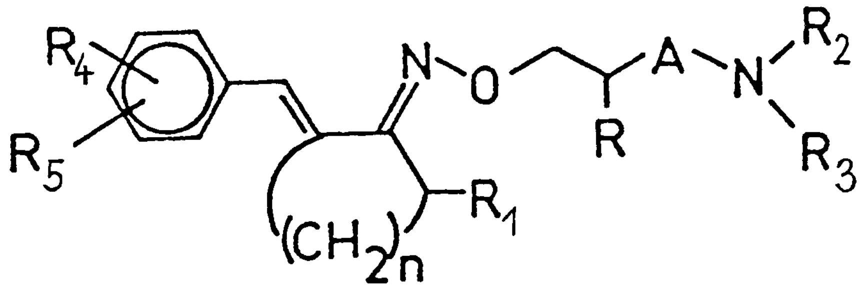

I ,

wherein

| | |
|---|---|
| R | represents hydrogen, $C_{1-4}$ alkyl or hydroxyl, |
| $R_1$ | stands for $C_{1-12}$ alkyl, |
| $R_2$ and $R_3$ | independently each represents hydrogen, a $C_{1-12}$ alkyl group, optionally substituted by hydroxy, or a $C_{2-12}$ alkenyl group, |
| | or |
| $R_2$ and $R_3$ | together with the nitrogen atom to which they are attached form a 4- to 7-membered heterocyclic ring, optionally comprising an oxygen, sulfur or a further nitrogen atom, which latter optionally may carry a phenyl, benzyl or $C_{1-4}$ alkyl substituent, |
| $R_4$ and $R_5$ | independently each stands for hydrogen, halogen or a $C_{1-4}$ alkoxy group, or together represent a 3,4-methylenedioxy group, |
| n | is an integer from 2 to 5 |
| | and |
| A | represents a valency bond or a $-CH_2$-group, |

as well as stereo and optically active isomers and their mixtures, acid-addition salts and quaternary ammonium derivatives thereof.

Furthermore the invention is concerned with a process for preparing these compounds as well as medicaments containing them and the use of them for preparing medicaments, particularly having ulcus and gastric-acid-secretion inhibiting effects.

The invention is concerned with novel trisubstituted cycloalkane derivatives with useful pharmacological, particularly ulcus and gastric-acid-secretion inhibiting, effects, a process for preparing them as well as medicaments containing these compounds and the use of them.

Some aminohydroxypropoxyimino derivatives have been known in the art.

The fluorene derivative "IPS-339" of formula

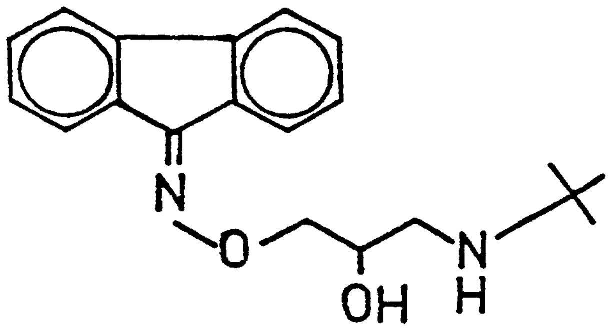

VI

and the methyl cyclopropylketone derivative of formula

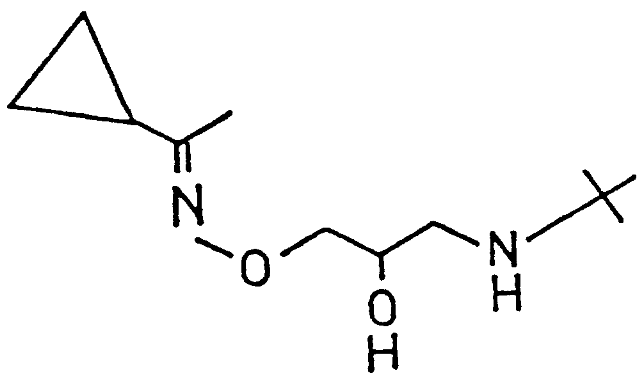

VII,

known as Falintolol, exhibit beta-adrenergic blocking activity.

Published PCT patent application 8,402,908 describes carbostyrylketoxime derivatives possessing beta-adrenergic blocking activity. These compounds are useful in the treatment of glaucoma.

Belgian patent specification 886,471 describes benzothiophene derivatives of general formula

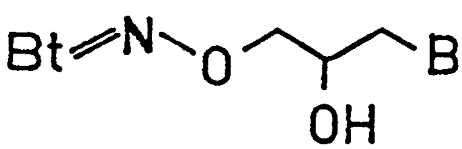

VIII,

wherein

Bt represents a benzothiophene group

and

B stands for a basic group containing a nitrogen atom.

Peraclopon of formula

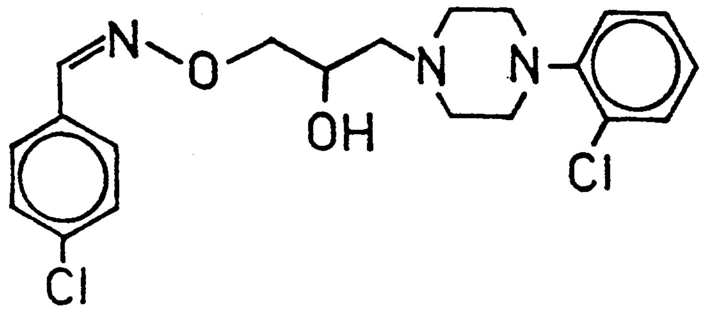

IX

is a lipid level lowering agent, and Peradoxime of the formula

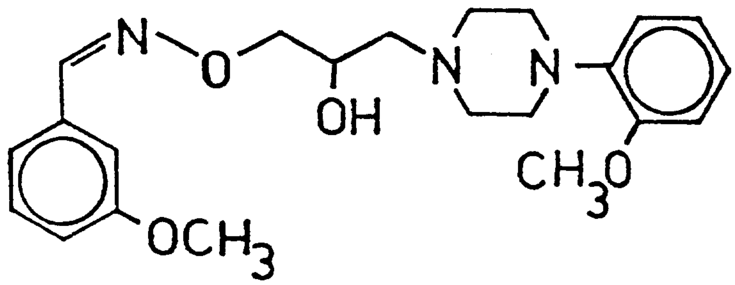

X

has a hypotensive effect.

US patent 4,652,586 relates to compounds of general formula VIII, wherein Bt is a fluorene group. The compounds reduce the inner pressure of eye and exhibit a selective $\beta_2$-adrenergic antagonist effect.

Published German patent application 4,027,052 relates to disubstituted cycloalkane derivatives of formula

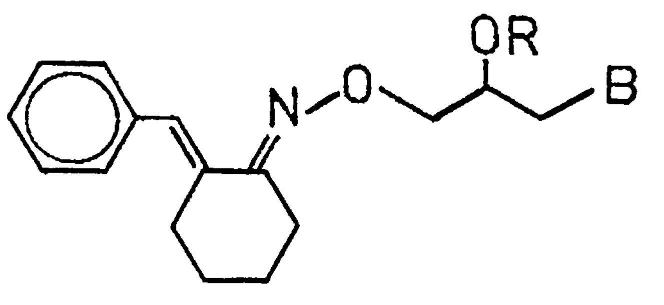

XI,

wherein R stands for hydrogen or benzoyl and B is a basic group containing a nitrogen atom, possessing primarily antiarrhythmic properties.

European patent 5,129 describes a new substituted benzimidazole derivative of formula

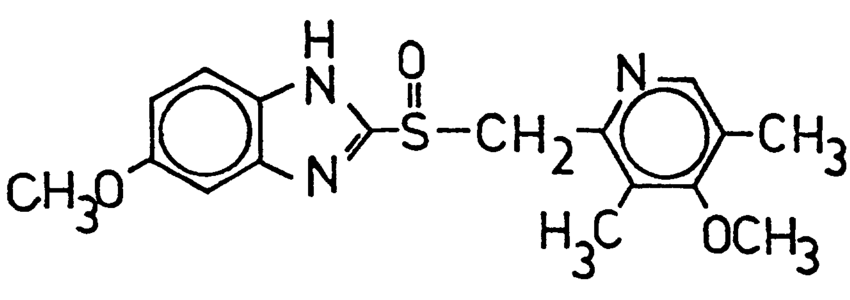

XII

which is the first representative of the compounds having $H^+/K^+$-ATP-ase inhibiting activity.

Hungarian patent 194,244 describes compounds of general formula

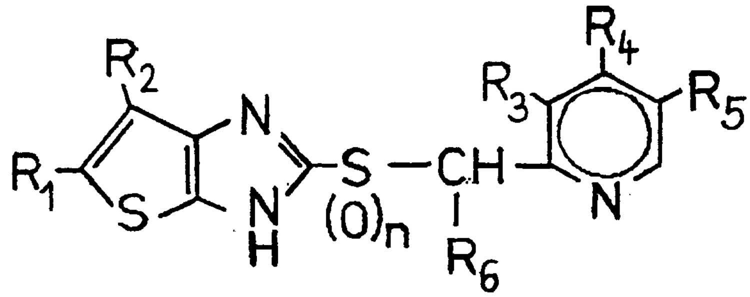

XIII,

wherein n is 0 or 1, $R_1$ , $R_2$ and $R_6$ each represents hydrogen, alkyl or alkoxy and $R_3$ , $R_4$ and $R_5$ each stands for halogen, alkyl or alkoxy, exerting $H^+/K^+$-ATP-ase inhibiting effect.

The problem underlying to the invention is to create novel trisubstituted cycloalkane derivatives showing valuable pharmacological properties, particularly ulcus and gastric-acid-secretion inhibiting effects, a process for preparing them as well as medicaments containing these compounds and the use of them.

Surprisingly this has been solved by the invention.

Thus a subject-matter of the invention are (phenylmethylene)-2-(alkyl)-1-[ω-(amino)-alkoxyimino]-cycloalkane derivatives of general formula

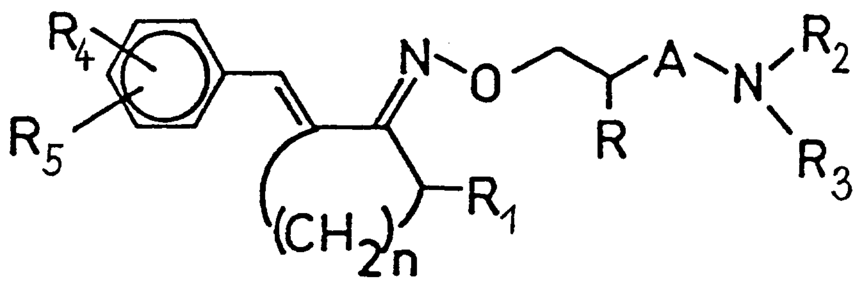

I,

wherein

| | |
|---|---|
| R | represents hydrogen, $C_{1-4}$ alkyl or hydroxyl, |
| $R_1$ | stands for $C_{1-12}$ alkyl, |
| $R_2$ and $R_3$ | independently each represents hydrogen, a $C_{1-12}$ alkyl group, optionally substituted by hydroxy, or a $C_{2-12}$ alkenyl group |
| | or |
| $R_2$ and $R_3$ | together with the nitrogen atom to which they are attached form a 4- to 7-membered heterocyclic ring, optionally comprising an oxygen, sulfur or a further nitrogen atom, which latter optionally may carry a phenyl, benzyl or $C_{1-4}$ alkyl substituent, |
| $R_4$ and $R_5$ | independently each stands for hydrogen, halogen or a $C_{1-4}$ alkoxy group or together represent a 3,4-methylenedioxy group, |
| n | is an integer from 2 to 5 |
| | and |
| A | represents a valency bond or a $-CH_2-$ group, |

as well as stereo and optically active isomers and their mixtures, acid-addition salts and quaternary ammonium derivatives thereof.

The chemical structure of the (phenylmethylene)-2-(alkyl)-1-[ω-(amino)-alkoxyimino]-cycloalkane derivatives according to the invention is basically different from that of the Prior Art compounds and the activity thereof is different from that of the latter ones and surprising.

The term "alkyl group" used throughout the specification relates to straight or branched chained saturated aliphatic hydrocarbon groups having the given number of carbon atom(s), e. g. methyl, ethyl, propyl, isopropyl, (methyl)-ethyl, n-butyl or tert.-butyl groups. The term "alkenyl group" designates straight or branched chained alkenyl groups containing the given number of carbon atoms, e. g. vinyl, allyl, 2-(methyl)-allyl, prop-1-enyl, but-1-enyl, but-2-enyl or hex-2-enyl groups. The term "alkoxy group" relates to

alkyl ether groups comprising 1 to 4 carbon atom(s), e. g. methoxy, ethoxy or tert.-butoxy groups. The term "halogen atom" encompasses all the four halogen atoms, that is fluorine, chlorine, bromine and iodine. The term "4- to 7-membered ring" relates to aromatic or partially or completely saturated heterocyclic groups which contain as [a] heteroatom(s) a nitrogen and optionally an oxygen, sulfur or further nitrogen atom, e. g. piperidyl, morpholinyl, piperazinyl, furyl, imidazolyl, pyridinyl, pyrazolyl, imidazolyl and hexamethyleneimino groups, and the latter further heteroatom optionally may carry a phenyl, benzyl or $C_{1-4}$ alkyl substituent.

It is preferred that the $C_{1-4}$ alkyl group which may be represented by R and/or the $C_{1-4}$ alkyl substituent by which the optional further nitrogen atom of the 4- to 7-membered heterocyclic ring which may be formed by $R_2$ and $R_3$ together with the nitrogen atom to which they are attached may be substituted and/or the $C_{1-4}$ alkoxy group(s) which may be represented by $R_4$ and/or $R_5$ is/are such having from 1 to 3, particularly 1 or 2, most particularly 1, carbon atom(s) and/or the $C_{1-12}$ alkyl group(s) which may be represented by $R_2$ and/or $R_3$ is/are such having from 1 to 8, particularly 1 to 4, more particularly 1 to 3, most particularly 1 or 2, above all 1, carbon atom(s) and/or the $C_{1-12}$ alkyl group which may be represented by $R_1$ is such having from 1 to 8, particularly 1 to 7, most particularly 1 or 3 to 7, carbon atom(s) and/or the $C_{2-12}$ alkenyl group(s) which may be represented by $R_2$ and/or $R_3$ is/are such having from 2 to 6, particularly 2 to 4, most particularly 2 or 3, above all 2, carbon atom(s) and/or the 4- to 7-membered heterocyclic ring which may be formed by $R_2$ and $R_3$ together with the nitrogen atom to which they are attached is such having from 5 to 7, particularly 5 or 6, carbon atoms, most particularly a morpholino, pyrrolidinyl, piperidinyl, piperazinyl or hexamethyleneimino ring, and/or the halogen atom(s) which may be represented by $R_4$ and/or $R_5$ is/are chlorine, fluorine and/or bromine, particularly the first one, and/or the integer represented by n is from 2 to 4, particularly 3 or 4, most particularly 3.

A particularly preferred group of compounds according to the invention are those in which

| | |
|---|---|
| $R_1$ | stands for $C_{1-8}$ alkyl, |
| $R_2$ and $R_3$ | independently each represents $C_{1-4}$ alkyl |
| | or |
| $R_2$ and $R_3$ | together with the nitrogen atom to which they are attached form a 5- or 6-membered heterocyclic ring, optionally comprising a further nitrogen atom, which latter optionally may carry a benzyl substituent, |
| $R_4$ and $R_5$ | independently each stands for hydrogen, halogen or methoxy, |
| n | is 3 or 4 |
| | and |
| A and R | are as above defined. |

Particularly preferred compounds according to the invention are (R,S)-6-(E)-[4'-(fluoro)-phenylmethylene]-2-[pentyl]-1-(E)-2''-{4'''-(phenylmethyl)-piperazin-1'''-yl}-ethoxyimino]-cyclohexane, (R,S)-2-[propyl]-7-(E)-[4'-(chloro)-phenylmethylene]-1-(E)-[2''-{hydroxy}-3''-(N-pyrrolidinyl)-propoxyimino]-cycloheptane, (R,S)-6-(E)-[4'-(fluoro)-phenylmethylene]-2-[methyl]-1-(E)-[3''-(dimethylamino)-2''-(methyl)-propoxyimino]-cyclohexane, (R,S)-6-(E)-[2'-(methoxy)-phenylmethylene]-2-[hexyl]-1-(E)-[2''-(diethylamino)-ethoxyimino]-cyclohexane, (R,S)-6-(E)-[4'-(fluoro)-phenylmethylene]-2-[heptyl]-1-(E)-[2''-(dimethylamino)-ethoxyimino]-cyclohexane, (R,S)-7-(E)-[4'-(chloro)-phenylmethylene]-2-[propyl]-1-(E)-[2''-(diethylamino)-ethoxyimino]-cycloheptane and (R,S)-6-(E)-[3',4'-di-(chloro)-phenylmethylene]-2-[methyl]-1-(E)-[2''-(diethylamino)-ethoxyimino]-cyclohexane as well as stereo and optically active isomers, acid-addition salts and quaternary ammonium derivatives thereof.

Suitably the acid-addition salts of the (phenylmethylene)-2-(alkyl)-1-[ω-(amino)-alkoxyimino]-cycloalkane derivatives of formula I according to the invention are pharmaceutically acceptable ones. Advantageous acid-addition salts are those with hydrogen halides, sulfuric acid, phosphoric acid, tartaric acid, succinic acid, acetic acid, fumaric acid, maleic acid, methanesulfonic acid or propionic acid.

The (phenylmethylene)-2-(alkyl)-1-[ω-(amino)-alkoxyimino]-cycloalkane derivatives of formula I according to the invention may comprise one or two asymmetric carbon atoms depending on the character of the substituents; thus they can be in optically active forms, too. The invention covers all of the racemic or optically active forms of the compounds of general formula I according to the invention.

According to a further aspect of the invention there is provided for a process for preparing the compounds according to the invention which is characterized in that in a manner known per se

a) (phenylmethylene)-2-(alkyl)-cycloalkane-1-one or -1-thione derivatives of general formula

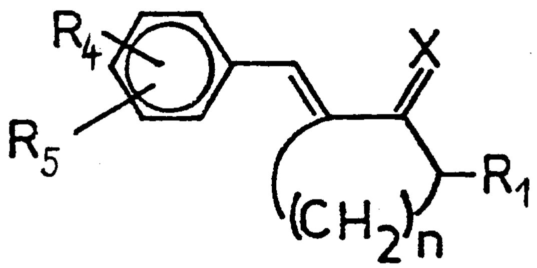

II,

wherein $R_1$, $R_4$, $R_5$ and n are as above defined and X stands for oxygen or sulfur, are reacted with substituted alkyl-0-hydroxylamines of general formula

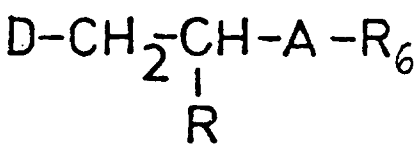

III,

wherein D represents a group of formula $H_2N$-0-, $R_6$ stands for a group of formula

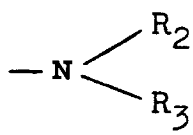

,

in which latter $R_2$ and $R_3$ are as above defined, and R and A are as above defined, or in the presence of a basic condensing agent with acid addition salts thereof

or

b) for preparing compounds of general formula I, wherein R represents hydrogen or $C_{1-4}$ alkyl and $R_1$, $R_2$, $R_3$, $R_4$, $R_5$, A and n are as above defined, (phenylmethylene)-2-(alkyl)-cycloalkane-1-oxime derivatives of general formula

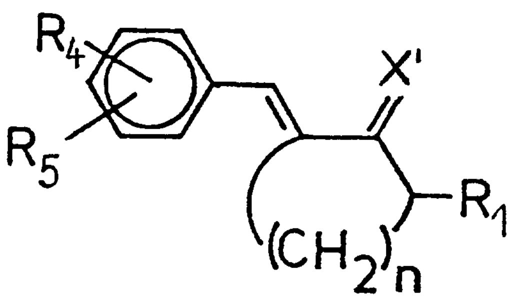

II' ,

wherein $R_1$, $R_4$, $R_5$ and n are as above defined and X' stands for a group of formula = N - OH, are reacted with substituted alkylhalides of general formula

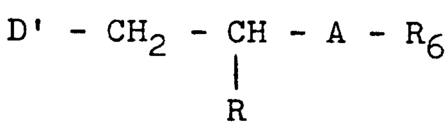

III',

wherein D' stands for halogen, R represents hydrogen or $C_{1-4}$ alkyl, $R_6$ is a group of formula

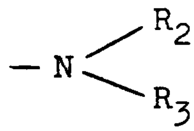 ,

in which latter $R_2$ and $R_3$ are as above defined, and A is as above defined, or with acid-addition salts thereof in the presence of a basic condensing agent,

or

c) for preparing compounds of general formula I, wherein R represents hydroxy, A stands for a group of formula - $CH_2$ - and $R_1$ , $R_2$ , $R_3$ , $R_4$ , $R_5$ and n are as above defined, (phenylmethylene)-2(alkyl)-cycloalkane-1-oxime derivatives of general formula II', wherein X' represents a group of formula = N - 0H and $R_1$ , $R_4$ , $R_5$ and n are as above defined, are reacted with 2,3-(epoxy)-propoxyhalides of general formula

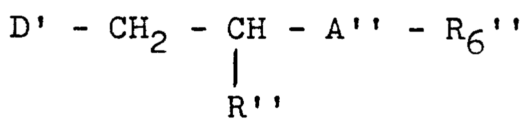 III'' ,

wherein D' represents halogen and A'', $R_6$'' and R'' together form a group of formula - $CH_2$ - 0 -, in the presence of a basic condensing agent, and the thus obtained (phenylmethylene)-2-(alkyl)-1-[2',3'-(epoxy)-propoxyimino]-cycloalkanes of general formula

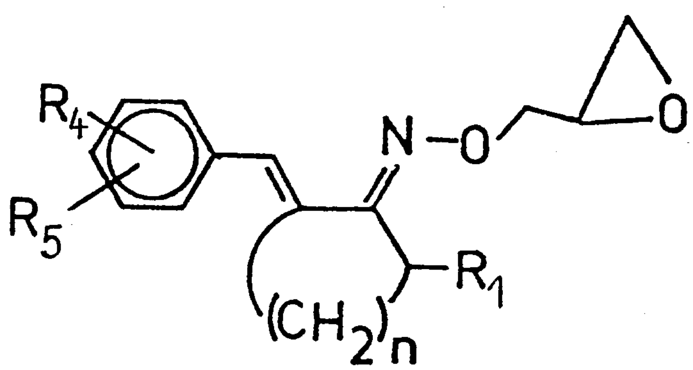 V,

wherein $R_1$ , $R_4$ , $R_5$ and n are as above defined, are reacted with amines of general formula

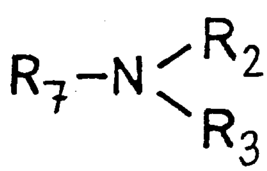 IV;

wherein $R_7$ stands for hydrogen, and $R_2$ and $R_3$ are as above defined, or in the presence of a basic agent with acid-addition salts of the former

or

d) for preparing compounds of general formula I wherein R represents hydrogen or $C_{1-4}$ alkyl and $R_1$ , $R_2$ , $R_3$ , $R_4$ , $R_5$ , A and n are as above defined, (phenylmethylene)-2-(alkyl)-cycloalkane-1-oxime derivatives of general formula II', wherein X' represents a group of formula = N - 0H and $R_1$ , $R_4$ , $R_5$ and n are as above defined, are reacted with $\alpha,\omega$-dihalogenalkanes of general formula

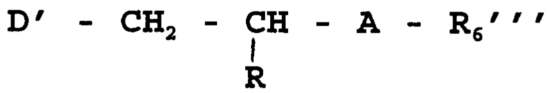

III''',

wherein D' stands for halogen, R represents hydrogen or $C_{1-4}$ alkyl, $R_6$''' means halogen and A is as above defined, in the presence of a basic condensing agent and the thus obtained (phenylmethylene)-2-(alkyl)-1-[ω-(halogen)-alkoxyimino]-cycloalkane derivatives of general formula

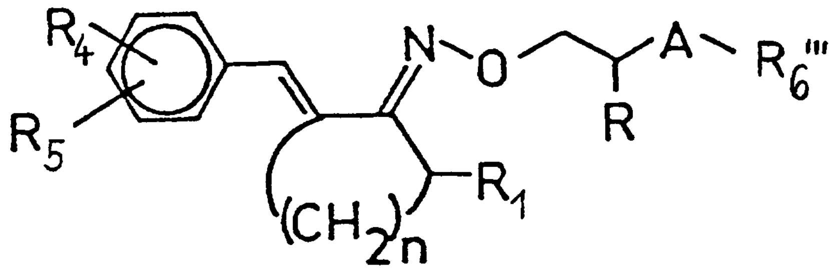

V',

wherein $R_1$ , $R_4$ , $R_5$ , A and n are as above defined, and $R_6$''' is as defined for formula III''', are reacted with amines of general formula

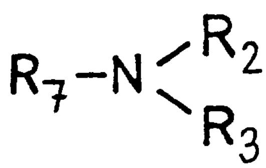

IV,

wherein $R_7$ stands for hydrogen, and $R_2$ and $R_3$ are as above defined, or in the presence of a basic agent with acid-addition salts of the former,

and optionally in a manner known per se (phenylmethylene)-2-(alkyl)-1-[ω-(amino)-alkoxy-imino]-cycloalkane derivatives of general formula I thus obtained are converted into acid-addition salts or quaternary ammonium derivatives thereof or salts of (phenylmethylene)-2-(alkyl)-1-[ω-(amino)-alkoxyimino]-cycloalkane derivatives of general formula I thus obtained are converted into the free (phenylmethylene)-2-(alkyl)-1-[ω-(amino)-alkoxyimino]-cycloalkane derivative bases and/or into other acid-addition salts and/or mixtures or racemates of the above compounds of formula I are separated to the stereo and/or optically active isomers of the above compounds of formula I or the optically active isomers of the above compounds of formula I are racemised.

The reaction of the (phenylmethylene)-2-(alkyl)-cycloalkane-1-one or -1-thione derivatives of general formula II with the substituted alkyl-0-hydroxylamines of general formula III of variant a) of the process according to the invention is preferably carried out in inert solvents or in mixtures of such solvents, particularly in an aliphatic alcohol, e. g. methanol or ethanol, pyridine or triethylamine or in a mixture thereof. As a basic condensing agent preferably an organic base, particularly pyridine, piperidine or morpholine or a mixture thereof is used. If the solvent is an organic base, it may serve as condensing agent, too.

In the reaction of the (phenylmethylene)-2-(alkyl)-cycloalkane-1-oxime derivatives of general formula II' with the substituted alkylhalides of general formula III' of variant b) of the process according to the invention as basic condensing agents preferably alkali amides, particularly sodium amide, alkali hydrides, particularly sodium hydride, alkali metals, alkali alcoholates or alkali hydroxides, particularly sodium hydroxide, or mixtures thereof, such as mixtures of sodium hydroxide and potassium hydroxyde in the ratio by weight of 9 : 1 to 1 : 9 are used. Preferably the reaction is carried out in inert solvents or mixtures of such solvents, particularly in an aliphatic or cyclic ether, e. g. diisopropylether, dibutylether, dioxane or tetrahydrofuran, aromatic hydrocarbon, e. g. benzene, toluene or xylene, dimethylformamide, dimethyl acetamide or dimethyl sulfoxide or in a mixture thereof.

Suitably the reaction of the (phenylmethylene)-2-(alkyl)-cycloalkane-1-oxime derivatives of general formula II' with the 2,3-(epoxy)-propoxyhalides of formula III'' or $\alpha,\omega$-dihalogenalkanes of general formula III''' respectively, of variant c) or d), respectively, of the process according to the invention is carried out in inert or relatively inert solvents. Preferably as inert solvents alcohols, particularly ethanol, or benzene, toluene, xylene or aliphatic cyclic ethers or dimethylformamide or mixtures thereof are used. As basic condensing agent preferably an alkali amide, particularly sodium amide or an alkali hydride, particularly sodium hydride, or an alkali alcoholate is used. Also alkali metals can be used as condensing agents. If an alkali hydroxide is used as a condensing agent, water may also serve as solvent (in such a case water is a relatively inert solvent in case of variant c), as on increasing the reaction time and temperature it goes into reaction with the epoxy compound).

Also the reaction of the (phenylmethylene)-2-(alkyl)-1-[2',3'-(epoxy)-propoxyimino]-cycloalkanes of general formula V with the amines of general formula IV of variant c) of the process according to the invention is preferably carried out in an inert solvent, e. g. in an alcohol, advantageously aliphatic alcohol, particularly ethanol, acetonitrile, or an aliphatic or cyclic ether, e. g. dioxane or tetrahydrofuran or a mixture thereof, but when using amines of general formula IV having high boiling point also it can be carried out without using any solvent. In such cases an excess of the applied amine serves as solvent. As basic agents applied in case of using acid-addition salts of the amines of formula IV preferably tri-$C_{1-4}$-alkylamines, such as triethylamine, are used. The reaction temperature may be varied within a wide interval. It can be room temperature to the boiling point of the reaction mixture an optimal rate of reaction being achieved at the boiling point of the reaction mixture. Analogous considerations apply for the second step of variant d) of the process according to the invention.

For converting the (phenylmethylene)-2(alkyl)-1-[$\omega$-(amino)-alkoxyimino]-cycloalkane derivatives of general formula I into acid-addition salts advantegeously hydrogen halides, sulfuric acid, phosphoric acid, tartaric acid, succinic acid, acetic acid, fumaric acid, maleic acid, methanesulfonic acid or propionic acid can be used. For preparing quaternary ammonium compounds the (phenylmethylene)-2-(alkyl)-1-[$\omega$-(amino)-alkoxyimino]-cycloalkane derivatives of general formula I can be reacted with reactants suitable for quaternarization, e. g. with alkyl halides.

If the compounds or intermediates having one or two asymmetric carbon atoms are prepared in the form of a diastereomeric mixture, they can be separated into racemic or optically active isomers in a manner known per se, e. g. by fractional distillation, crystallization, chromatography or by forming diastereomeric salts with the aid of optically active acids such as tartaric acid, dibenzoyltartaric acid or camphorsulfonic acid.

The (phenylmethylene)-2-(alkyl)-cycloalkane-1-one or -1-thione derivatives of general formula II usable as starting substances are known and can be prepared e. g. according to the method described in J. Chem. Soc. [1955], 1 126 or in J. Am. Chem. Soc. 77 [1955], 624. The compounds of general formula III, wherein R is hydroxy, usable as starting substances can be prepared as described in J. Pharm. Sci. 58 [1969], 138. Thus the substituted alkyl-0-hydroxylamines of general formula III can be prepared as described in J. Pharm. Sci. 58 [1969], 138.

The (phenylmethylene)-2-(alkyl)-cycloalkane-1-oxime derivatives of general formula II' usable as starting compounds for variants b), c) and d) of the process according to the invention can be prepared e. g. according to the method described in Org. Synth. Coll. Vol. II [1943], 70.

The substituted alkylhalides of general formula III', the 2,3-(epoxy)-propoxyhalides of general formula III'' and the $\alpha,\omega$-dihalogenalkanes of general formula III''' usable as starting substances for variants b), c) and d) of the process according to the invention can be prepared as described in Helv. Chim. Acta 41 [1958], 1 072 to 1 108 or in Beilstein 17, 1/v 20.

As already mentioned, the (phenylmethylene)-2-(alkyl)-1-[$\omega$-(amino)-alkoxyimino]-cycloalkane derivatives according to the invention possess a considerable gastric-acid-secretion and ulcus inhibiting activity. They are also potent inhibitors of the gastric $H^+K^+$-ATP-ase. At the same time their toxicity is slight, so that they can be used as active ingredients in pharmaceutical compositions.

Hence by the invention also there are provided for medicaments which are characterized in that they contain as [an] active ingredient(s) at least one compound according to the invention, suitably in admixture with at least one inert solid and/or liquid pharmaceutical carrier and/or diluent and/or excipient.

The medicaments according to the invention can be in the form of pharmaceutical preparations. These can be prepared by methods known per se and thus suitably by 1 or more (phenylmethylene)-2-(alkyl)-1-[$\omega$-(amino)-alkoxyimino]-cycloalkane derivatives according to the invention with 1 or more inert solid and/or liquid carrier(s) and/or diluent(s) and/or excipient(s) and bringing the mixture to a galenic form.

Preferably the pharmaceutical preparations of the invention are in the form of tablets or dragées for oral administration. The daily dose of the (phenylmethylene)-2-(alkyl)-1-[$\omega$-(amino)-alkoxyimino]-cycloalkane de-

rivatives according to the invention can vary within wide ranges depending on several factors, e. g. on the activity of the active ingredient(s), the patient's condition and age and the severity of the disease. The oral dose is generally 1 to 300 mg/day. It has to be stressed that these doses are only of informative character and the administered dose must always be determined by the physician therapeutist.

The (phenylmethylene)-2-(alkyl)-1-[ω-(amino)-alkoxyimino]-cycloalkane derivatives according to the invention also can be in the form of solution or suspension preparations.

The (phenylmethylene)-2-(alkyl)-1-[ω-(amino)-alkoxyimino]-cycloalkane derivatives according to the invention can serve as active ingredients for preparing medicaments having ulcus and gastric-acid-secretion inhibiting effects, for example, useful in the treatment of disorders caused by hyperacidity (gastric or duodenal ulcer), in the treatment of gastric mucosa caused by antiphlogistics (glucocorticoids, salicylic acid derivatives) or for the mitigation of gastric disorders caused by alcoholism.

Hence a further subject-matter of the invention is the use of the compounds according to the invention for preparing medicaments, particularly having ulcus and gastric-acid-secretion inhibiting effects.

The compounds according to the invention can be used administering to the patient an effective amount of them.

The biological activity of the (phenylmethylene)-2-(alkyl)-1-[ω-(amino)-alkoxyimino]-cycloalkane derivatives according to the invention is shown by the following tests.

I. Toxicity

The test was carried out according to the method of Lichtfield and Wilcoxon (Lichtfield, J.T. and Wilcoxon, F.W.: J. Pharmacol. Exp. Ther., 96, 99 [1949]) by using white mice belonging to the CFLP strain and weighing 10 to 22 g, 10 animals per dose. The test compounds were administered orally in a volume of 20 $cm^3$/kg. After treatment the animals were observed for a period of 14 days. The results are summarized in Table I.

<u>Table I</u>

Toxicity on mice

| Compound | | $LD_{50}$ mg/kg | |
|---|---|---|---|
| Designation | Example | i.p. | p.o. |
| (R,S)-6-(E)-[4'-(Fluoro)-phenylmethylene]-2-[pentyl]-1--(E)-[2"-{4'''-(phenylmethyl)-piperazin-1'''-yl}-ethoxyimino]-cyclohexane | 30 | > 300 | > 1 000 |
| (R,S)-2-[Propyl]-7-(E)-[4'-(Chloro)-phenylmethylene]-1--(E)-[2"-{hydroxy}-3"-{(N-pyrrolidinyl)-propoxyimino}]--cycloheptane | 42 | 100 to 300 | > 1 000 |
| (R,S)-6-(E)-[4'-(Fluoro)-phenylmethylene]-2-[methyl]-1--(E)-[3"-(dimethylamino)-2"-(methyl)-propoxyimino]-cyclohexane | 9 | about 300 | 600 |
| (R,S)-6-(E)-[2'-(Methoxy)-phenylmethylene]-2-[hexyl]-1--(E)-[2"-(diethylamino)-ethoxyimino]-cyclohexane | 33 | 88 | about 1 000 |
| (R,S)-6-(E)-[4'-(Fluoro)-phenylmethylene]-2-[heptyl]-1--(E)-[2"-(dimethylamino)-ethoxyimino]-cyclohexane | 34 | 100 to 300 | > 1 000 |
| (R,S)-7-(E)-[4'-(Chloro)-phenylmethylene]-2-[propyl]-1--(E)-[2"-(diethylamino)-ethoxyimino]-cycloheptane | 38 | 100 to 300 | > 1 000 |
| (R,S)-6-(E)-[3',4'-Di-(chloro)-phenylmethylene]-2--[methyl]-1-(E)-[2"-(diethylamino)-ethoxyimino]-cyclohexane | 39 | 100 to 300 | > 1 000 |

## II. Gastric ulcer inhibiting effect

Test methods:

1. $H^+/K^+$-ATP-ase inhibition on pig's stomach. The test was carried out according to the method of Rabon and Sachs [E.C. Rabon, W.B.I. and G. Sachs: Preparation of gastric $H^+/K^+$-ATP-ase. Methods in Enzymology, **157**, 649-651 (1988)]. The activity of the prepared enzyme was measured both in the presence and in the absence of $K^+$ ions. The difference in the liberation of phosphorus representing the activity of the enzyme was measured.
2. The gastric-acid-secretion test was carried out on rats according to the method of Shay et al. [Shay, H., Komarov, S.A., Fels, S.S., Meranze, D., Gruenstein, M., Siplet, H.: Gastroenterology 5, 43-61 (1945)]. The liberated gastric acid content was determined by titration 4 hours after the ligature of the duodenum.
3. The cytoprotective effect was determined on rats according to the method of Robert [Robert, A.: Cytoprotection by prostaglandins. Gastroenterology **77**, 761-767 (1979)]. Rats weighing 200 to 250 g were used as test animals. 1 $cm^3$ of abs. ethanol was introduced to the stomach to produce an erosion of the stomach wall. The length and frequency of the lesions (erosion index and frequency) were measured and the percentage inhibition of lesion formation compared with vehicle-treated animals was calculated.
4. Measurement of $^{14}C$-aminopyrine accumulation by parietal cells. Gastric mucosal cells were prepared from rat stomach. Wistar rats (130-160 g) were killed by decapitation, the stomachs were rapidly excised and their contents were washed out with saline. The stomachs were then everted and filled with 2.5 mg/ml of pronase®-containing buffer. These sacs were incubated for 60 minutes at 37 °C in carbogen-gassed medium. This incubation was followed by gentle stirring at room temperature for 45 minutes by a magnetic stirrer in order to disperse the cells from the mucosa of the everted stomachs digested only from the serosal side. The viability of the cells was determined by trypan-blue exclusion test. The percentage of the parietal cells was determined on the basis of their morphological characteristics.

Acid production of the cells prepared in this way could be induced by cyclic AMP, histamine (in the presence of 3-isobutyl-1-methylxanthine) or carbachol. The acid production was assessed by measuring the accumulation of $^{14}C$-aminopyrine. The undissociated weak base can penetrate into the acid-containing compartments of the cells. In the acidic compartment the aminopyrine dissociates and for the dissociated form the membrane is impermeable. Thus, the distribution of $^{14}C$-aminopyrine between the extracellular and intracellular spaces is an indirect quantitative index for the cellular acid production [W. Schepp, J. Schmidtler, C. Tatge, V. Schusdziarra and M. Classen: Am. J. Physiol. 259 (Gastrointest. Liver Physiol. 22) G646-G654 (1990)].

## Results

1. The compounds according to the invention are potent inhibitors of the $H^+/K^+$-ATP-ase (proton pump), the $IC_{50}$ values are between 5 and > 30 $\mu$M. The compounds inhibit the induced acid production on isolated parietal cells (inhibition of $^{14}C$-aminopyrine accumulation) in low concentration.

Table II

Inhibition of $H^+K^+$-ATP-ase on partially purified pig's microsome specimen

| Compound: Designation | Compound: Example | $IC_{50}$ (μM) |
|---|---|---|
| (R,S)-6-(E)-[4'-(Fluoro)-phenylmethylene]-2-[pentyl]-1--(E)-[2"-{4'''-(phenylmethyl)-piperazin-1'''-yl}-ethoxyimino]-cyclohexane | 30 | > 30 |
| (R,S)-2-[Propyl]-7-(E)-[4'-(chloro)-phenylmethylene]-1--(E)-[2"-{hydroxy}-3"-{(N-pyrrolidinyl)-propoxyimino}]--cycloheptane | 42 | 10 |
| (R,S)-6-(E)-[4'-(Fluoro)-phenylmethylene]-2-[methyl]-1--(E)-[3"-(dimethylamino)-2"-(methyl)-propoxyimino]-cyclohexane | 9 | > 30 |
| (R,S)-6-(E)-[2'-(Methoxy)-phenylmethylene]-2-[hexyl]-1--(E)-[2"-(diethylamino)-ethoxyimino]-cyclohexane | 33 | 5 |
| (R,S)-6-(E)-[4'-(Fluoro)-phenylmethylene]-2-[heptyl]-1--(E)-[2"-(dimethylamino)-ethoxyimino]-cyclohexane | 34 | 13 |
| (R,S)-7-(E)-[4'-(Chloro)-phenylmethylene]-2-[propyl]-1--(E)-[2"-(diethylamino)-ethoxyimino]-cycloheptane | 38 | 7 |
| (R,S)-6-(E)-[3',4'-Di-(chloro)-phenylmethylene]-2--[methyl]-1-(E)-[2"-(diethylamino)-ethoxyimino]-cyclohexane | 39 | 17 |

2. Considering the acid-secretion inhibiting effect the $ED_{50}$ values of the most effective compounds according to the invention are 1.1 and 5.4 mg/kg (when administered intraduodenally). This proves that the compounds are favourable acid-secretion inhibitors in vivo.

3. The cytoprotective effect of the compounds is significant, and according to the literature (D.E. Wilson: Therapeutic aspects of prostaglandins in the treatment of peptic ulcer disease. Dig. Dis. Sci. 1986. 31, 42-46S) this is a favourable characteristic considering the potential therapeutic utility.

Table III

Gastric-acid-secretion inhibiting and cytoprotective effects

| Compound: Designation | Example | Acid-secretion inhibition, $ED_{50}$ mg/kg, p.o. | Ethanol erosion $ED_{50}$ mg/kg,p.o. | Ratio of gastric-acid-secretion inhibition to erosion inhibition |
|---|---|---|---|---|
| (R,S)-6-(E)-[4'-(Fluoro)-phenylmethylene]-2-[pentyl]-1-(E)-[2"-{4'''-(phenylmethyl)-piperazin-1'''-yl}-ethoxyimino]-cyclohexane | 30 | 200 | 3.9 | 51 |
| (R,S)-2-[Propyl]-7-(E)-[4'-(Chloro)-phenylmethylene]-1-(E)-[2"-{hydroxy}-3"-{(N-pyrrolidinyl)-propoxyimino}]-cycloheptane | 42 | 50 to 200 | 1.6 | 31 to 125 |
| (R,S)-6-(E)-[4'-(Fluoro)-phenylmethylene]-2-[methyl]-1-(E)-[3"-(dimethylamino)-2"-(methyl)-propoxyimino]-cyclohexane | 9 | 30 to 120 | 1.2 | 25 to 100 |
| (R,S)-6-(E)-[2'-(Methoxy)-phenylmethylene]-2-[hexyl]-1-(E)-[2"-(diethylamino)-ethoxyimino]-cyclohexane | 33 | 102.7 | 1.4 | 73.4 |
| (R,S)-6-(E)-[4'-(Fluoro)-phenylmethylene]-2-[heptyl]-1-(E)-[2"-(dimethylamino)-ethoxyimino]-cyclohexane | 34 | 200 | 2.9 | 69 |
| (R,S)-7-(E)-[4'-(Chloro)-phenylmethylene]-2-[propyl]-1-(E)-[2"-(diethylamino)-ethoxyimino]-cycloheptane | 38 | 52.9 | 2.6 | 20.3 |
| 5-Methoxy-2-(4-methoxy-3,5-dimethyl-2-pyridylmethyl-sulfinyl)-benzimidazole [Omeprazole] | Reference substance A | 3.9 | 4.5 | 0.9 |

Table III continued

| Compound – Designation | Compound – Example | Acid-secretion inhibition, $ED_{50}$ mg/kg, p.o. | Ethanol erosion $ED_{50}$ mg/kg,p.o. | Ratio of gastric-acid-secretion inhibition to erosion inhibition |
|---|---|---|---|---|
| 1-Cyan-2-methyl-3-[2-(5-methyl-4-imidazolyl)-methyl-thioethyl]-guanidine [Cimetidine] | Reference substance B | 59.1 | 100 to 200 | 0.3 to 0.6 |
| 11-[(4-Methyl-1-piperazinyl)-acetyl]-5H-pyrido[2,3-b][1,4]benzodiazepin-6(11H)-one [Pirenzepine] | Reference substance C | 7.9 | 18.6 | 0.4 |
| Basic aluminium salt of saccharose-hydrogensulfate [Sucralfate] | Reference substance D | - | 69.0 | - |

From the above test results it can be established that the compounds according to the invention are only slightly toxic and at the same time they inhibit the gastric-acid secretion at doses 5 to 30 times lower than the toxic doses ($LD_{50}$). From the low $ED_{50}$ values obtained in the ethanol erosion test it can be seen that the cytoprotective effect of the test compounds according to the invention is highly superior to their gastric-acid-secretion activity. The compounds according to the invention are somewhat less potent

inhibitors of the gastric-acid-secretion than omeprazole or pirenzepine, but considering the inhibition of the erosion of the stomach wall produced by ethanol they are superior to both reference substances. From this fact it appears that the mechanism of the effect of the compounds according to the invention is different from that of the known substances exerting an antiulcer activity. The difference demonstrated by the gastric-acid-secretion inhibition/erosion inhibition ratios is very favourable, especially in the treatment of human diseases wherein the injury of the stomach wall occurs simultaneously with a decreased acid production (e.g. gastric disorders caused by alcoholism).

Summarizing what has been said it can be established that the compounds according to the invention inhibit the enzyme responsible for the acid production in low concentrations. They are also effective in vivo and exhibit a gastric-acid-secretion inhibiting activity at doses considerably lower than the acute toxic doses. Their cyto-protective activity is excellent. Accordingly, proton-pump-inhibiting and cytoprotective substances have been found, which

a) are chemically different from the hitherto known molecules possessing similar activities, so that such effects could not be aforeseen on the basis of the chemical structure,

b) possess an enzyme-inhibiting activity being of $\mu$M order of magnitude under the experimental conditions used and

c) have an outstanding cytoprotective effect independent of the proton-pump-inhibiting activity.

The invention is further illustrated by the following Examples. In the thin layer chromatography all ratios indicated are by volume.

**Example 1**

**(R,S)-6-(E)-Phenylmethylene-2-methyl-1-(E)-[3-dimethylamino)-propoxyimino]-cyclohexane**

6-(E)-Phenylmethylene-2-methylcyclohexan-1-one-(E)-oxime (21.53 g; 0.1 mole) is transformed into a salt with the aid of sodium hydride (4.8 g; 0.1 mole, 50% by weight oily dispersion) in the mixture of 30 $cm^3$ of dimethylformamide and 20 $cm^3$ of benzene, and the salt thus obtained is reacted with 3-chloro-N,N-dimethylpropylamine (13.38 g; 0.11 mole) at the boiling point of the mixture. The boiling is continued until the starting oxime cannot be detected in the reaction mixture by thin layer chromatography (benzene:methanol 4:1, Kieselgel 60 $F_{254}$).
Yield: 27.0 g (89.7 %).
(E)-2-Butenedioate (1/1) M.p.: 116 to 118.5 °C.

| Analysis for the formula $C_{23}H_{32}N_2O_5$ (416.51): | | | |
|---|---|---|---|
| Calculated:<br>Found: | C % = 66.32<br>C % = 66.55 | H % = 7.75<br>H % = 7.74 | N % = 6.73<br>N % = 6.60. |

UV: $\lambda_{max}$ = 273 nm ($\epsilon$ = 17469).

**Example 2**

**(R,S)-6-(E)-Phenylmethylene-2-methyl-1-(E)-[3-(dimethylamino)-2-methylpropoxyimino]-cyclohexane**

One proceeds as specified in Example 1 except that instead of 3-chloro-N,N-dimethylpropylamine 3-chloro-2,N,N-trimethylpropylamine (14.92 g; 0.11 mole) is used.
Yield: 27.82 g (88.60 %).
(E)-2-Butenedioate (1/1) M.p.: 162 to 167 °C.

| Analysis for the formula $C_{24}H_{34}N_2O_5$ (430.53): | | | |
|---|---|---|---|
| Calculated:<br>Found: | C % = 66.95<br>C % = 66.35 | H % = 7.96<br>H % = 7.96 | N % = 6.51<br>N % = 6.53. |

UV: $\lambda_{max}$ = 273 nm ($\epsilon$ = 16663).

**Example 3**

**(R,S)-6-(E)-Phenylmethylene-2-methyl-1-(E)-(3-morpholino-propoxyimino)-cyclohexane**

One proceeds as specified in Example 1 except that instead of 3-chloro-N,N-dimethylpropylamine N-(3-chloropropyl)-morpholine(18.00 g; 0.11 mole) is used.
Yield: 31.23 g (91.19 %).
(E)-2-Butenedioate (1/1) M.p.: 146 to 150 °C.

| Analysis for the formula $C_{25}H_{34}N_2O_6$ (458.54): | | | |
|---|---|---|---|
| Calculated: | C % = 65.47 | H % = 7.47 | N % = 6.11 |
| Found: | C % = 65.20 | H % = 7.50 | N % = 6.34. |

UV: $\lambda_{max}$ = 270 nm ($\epsilon$ = 16019).

**Example 4**

**(R,S)-6-(E)-Phenylmethylene-2-methyl-1-(E)-(2-pyrrolidinyl-ethoxyimino)-cyclohexane**

One proceeds as specified in Example 1 except that instead of 3-chloro-N,N-dimethylpropylamine N-(2-chloroethyl)-pyrrolidine(14.7 g; 0.11 mole) is used.
Yield: 19.86 g (63.56 %).
(E)-2-Butenedioate (1/1) M.p.: 140 to 146 °C.

| Analysis for the formula $C_{24}H_{32}N_2O_5$ (428.52): | | | |
|---|---|---|---|
| Calculated: | C % = 67.26 | H % = 7.53 | N % = 6.54 |
| Found: | C % = 66.87 | H % = 7.50 | N % = 6.30. |

UV: $\lambda_{max}$ = 270 nm ($\epsilon$ = 32776).

**Example 5**

**(R,S)-6-(E)-Phenylmethylene-2-methyl-1-(E)-(2-piperidinyl-ethoxyimino)-cyclohexane**

One proceeds as specified in Example 1 except that instead of 3-chloro-N,N-dimethylpropylamine N-(2-chloroethyl)-piperidine (16.24 g; 0.11 mole) is used.
Yield: 32.32 g (99.01 %).
(E)-2-Butenedioate (1/1) M.p.: 170.5 to 175.5 °C.

| Analysis for the formula $C_{25}H_{34}N_2O_5$ (442.54): | | | |
|---|---|---|---|
| Calculated: | C % = 67.85 | H % = 7.74 | N % = 6.33 |
| Found: | C % = 66.64 | H % = 7.65 | N % = 6.22 |

UV: $\lambda_{max}$ = 270 nm ($\epsilon$ = 17753).

**Example 6**

**(R,S)-6-(E)-Phenylmethylene-2-methyl-1-(E)-[2-hexahydro-1H-azepinyl)-ethoxyimino]-cyclohexane**

One proceeds as specified in Example 1 except that instead of 3-chloro-N,N-dimethylpropylamine N-(2-chloroethyl)-hexahydro-1H-azepine (17.78 g; 0.11 mole) is used.
Yield: 31.7 g (93.11 %).
(E)-2-Butenedioate (2/1) M.p.: 144 to 150 °C.

| Analysis for the formula $C_{24}H_{34}N_2O_3$ (426.55): | | | |
|---|---|---|---|
| Calculated:<br>Found: | C % = 67.57<br>C % = 67.22 | H % = 8.03<br>H % = 8.09 | N % = 6.67<br>N % = 6.63. |

UV: $\lambda_{max}$ = 266 nm ($\epsilon$ = 14757).

**Example 7**

**(R,S)-6-(E)-Phenylmethylene-2-methyl-1-(E)-[3-(dimethylamino)-2-hydroxypropoxyimino]-cyclohexane**

a) 6-(E)-Phenylmethylene-2-methylcyclohexan-1-one-(E)-oxime (21.53 g; 0.1 mole) is transformed into a salt with sodium hydride (4.8 g; 0.1 mole, 50% by weight oily dispersion) in the mixture of dimethylformamide and benzene, and the salt thus obtained is reacted with 1-chloro-2,3-epoxypropane (10.18 g; 0.11 mole) at a temperature between 40 °C and 50 °C. The stirring is continued until the starting oxime cannot be detected in the reaction mixture by thin layer chromatography (Kieselgel 60 $F_{254}$, n-hexane:dioxane 4:1). The reaction mixture is washed three times with 50 $cm^3$ of water each and the solvent is distilled off.
Yield: 27.03 g (99.61 %).
b) To a solution of the product obtained according to the method described in paragraph a) (27.03 g; 0.099 mole) and dimethylamine hydrochloride (9.87 g; 0.12 mole) in ethanol triethylamine (12.14 g; 0.12 mole) is dropped and the mixture is slowly heated to the boiling point. The boiling is continued until the starting oxime cannot be detected in the reaction mixture by thin layer chromatography (benzene:methanol 4:1, Kiselgel 60 $F_{254}$).
Yield: 18.36 g (58.02 %).
(E)-2-Butenedioate (2/1) M.p.: 126 to 149 °C.

| Analysis for the formula $C_{42}H_{60}N_4O_8$ (748,94): | | | |
|---|---|---|---|
| Calculated:<br>Found: | C % = 67.35<br>C % = 67.31 | H % = 8.08<br>H % = 8.10 | N % = 7.48<br>N % = 7.40. |

UV: $\lambda_{max}$ = 279 nm ($\epsilon$ = 31713).

**Example 8**

**(R,S)-6-(E)-(4-Fluorophenylmethylene)-2-methyl-1-(E)-(2-morpholinoethoxyimino)-cyclohexane**

One proceeds as specified in Example 1 except that instead of 6-(E)-phenylmethylene-2-methylcyclohexan-1-one-(E)-oxime 6-(E)-(4-fluorophenylmethylene)-2-methylcyclohexan-1-one-(E)-oxime (23.32 g; 0.1 mole) and instead of 3-chloro-N,N-dimethylpropylamine 4-(2-chloroethyl)-morpholine (16.46 g; 0.11 mole) is used.
Yield: 18.90 g (54.54 %).
(E)-2-Butenedioate (1/1) M.p.: 139 to 145 °C.

| Analysis for the formula $C_{24}H_{31}FN_2O_6$ (462.51): | | | | |
|---|---|---|---|---|
| Calculated:<br>Found: | C % = 62.32<br>C % = 61.72 | H % = 6.76<br>H % = 6.71 | N % = 6.06<br>N % = 6.07 | F % = 4.11<br>F % = 4.00. |

UV: $\lambda_{max}$ = 270 nm ($\epsilon$ = 16751).

**Example 9**

**(R,S)-6-(E)-(4-Fluorophenylmethylene)-2-methyl-1-(E)-[3-(dimethylamino)-2-methylpropoxyimino]-cyclohexane**

One proceeds as specified in Example 8 except that instead of 4-(2-chloroethyl)-morpholine 3-chloro-2,N,N-trimethylpropylamine (14.92 g; 0.11 mole) is used.
Yield: 32.59 g (98.03 %).
(E)-2-Butenedioate (1/1) M.p.: 188 °C.

| Analysis for the formula $C_{24}H_{33}FN_2O_5$ (448.52): | | | | |
|---|---|---|---|---|
| Calculated:<br>Found: | C % = 64.26<br>C % = 64.55 | H % = 7.41<br>H % = 7.43 | N % = 6.25<br>N % = 6.47 | F % = 4.24<br>F % = 4.20. |

UV: $\lambda_{max}$ = 290 nm ($\epsilon$ = 1611).

**Example 10**

**(R,S)-6-(E)-(4-Chlorophenylmethylene)-2-methyl-1-(E)-{2-[bis-(1-methylethyl)-amino]-ethoxyimino}-cyclohexane**

6-(E)-(4-Chlorophenylmethylene)-2-methylcyclohexan-1-one-(E)-oxime (24.95 g; 0.1 mole) is transformed into a salt with freshly prepared sodium methylate (5.4 g; 0.1 mole), and this salt is reacted with N-(2-chloroethyl)-N-(1-methylethyl)-2-propylamine (18.01 g; 0.11 mole) in dimethylformamide. Further on the process of Example 1 is followed.
Yield: 33.30 g (88.45 %).
(E)-2-Butenedioate (1/1) M.p.: 133 to 138 °C.

| Analysis for the formula $C_{26}H_{37}ClN_2O_5$ (493.04): | | | | |
|---|---|---|---|---|
| Calculated:<br>Found: | C % = 63.33<br>C % = 63.20 | H % = 7.56<br>H % = 7.71 | N % = 5.68<br>N % = 5.95 | Cl % = 7.19<br>Cl % = 7.21. |

UV: $\lambda_{max}$ = 276 nm ($\epsilon$ = 19782).

**Example 11**

**(R,S)-6-(E)-(4-Chlorophenylmethylene)-2-methyl-1-(E)-[3-(4-methyl-1-piperazinyl)-propoxyimino]-cyclohexane**

6-(E)-(4-Chlorophenylmethylene)-2-methyl-1-(E)-cyclohexan-1-one-(E)-oxime (24.95 g; 0.1 mole) is transformed into a salt with sodium hydride (4.8 g; 0.1 mole, 50% by weigth oily dispersion) in the mixture of 30 $cm^3$ dimethylformamide and 20 $cm^3$ benzene, and the salt thus obtained is reacted with 1-bromo-3-chloropropane (15.74 g; 0,1 mole) at a temperature of 60 °C until the starting oxime cannot be detected any more in the reaction mixture. Then it is cooled, washed three times with 50 $cm^3$ of water each and evaporated in vacuo. The thus-obtained 6-(E)-(4-chlorophenylmethylene)-2-methyl-1-(E)-(3-chloropropoxyimino)-cyclohexane (29.12 g; 89.52 %) is reacted with N-methylpiperazine (10.01 g; 0.1 mole) in ethanol at the boiling point of the reaction mixture.
Yield: 21.65 g (55.52 %) M.p.: 88 to 91 °C.
(E)-2-Butenedioate (1/2) M.p.: 221 °C (decomp.).

| Analysis for the formula $C_{30}H_{40}ClN_3O_9$ (622.1): | | | | |
|---|---|---|---|---|
| Calculated:<br>Found: | C % = 57.92<br>C % = 58.16 | H % = 6.48<br>H % = 6.30 | N % = 6.75<br>N % = 6.84 | Cl % = 5.70<br>Cl % = 5.63. |

UV: $\lambda_{max}$ = 270 nm ($\epsilon$ = 19408).

**Example 12**

**(R,S)-6-(E)-(4-Chlorophenylmethylene)-2-methyl-1-(E)-[3-(dimethylamino)-2-methylpropoxyimino]-cyolohexane**

One proceeds as specified in Example 10 except that instead of N-(2-chloroethyl)-N-(1-methylethyl)-2-propylamine 3-chloro-2,N,N-trimethylpropylamine (14.92 g; 0.11 mole) is used.
Yield: 28.15 g (80.68 %).
(E)-2-Butenedioate (1/1) M.p.: 184 to 188.5 °C.

| Analysis for the formula $C_{24}H_{33}ClN_2O_5$ (464.977): | | | | |
|---|---|---|---|---|
| Calculated:<br>Found: | C % = 61.99<br>C % = 60.52 | H % = 7.15<br>H % = 7.01 | N % = 6.03<br>N % = 5.93 | Cl % = 7.63<br>Cl % = 7.54. |

UV: $\lambda_{max}$ = 267 nm ($\epsilon$ = 19242).

**Example 13**

**(R,S)-6-(E)-(4-Bromophenylmethylene)-2-methyl-1-(E)-(3-morpholinopropoxyimino)-cyclohexane**

One proceeds as specified in Example 1 except that instead of 6-(E)-phenylmethylene-2-methylcyclohexan-1-one-(E)-oxime 6-(E)-(4-bromophenylmethylene)-2-methylcyclohexan-1-one-(E)-oxime (29.42 g; 0.1 mole) and instead of 3-chloro-N,N-dimethylpropylamine 4-(3-chloropropyl)-morpholine (18.00 g; 0.11 mole) is used.
Yield: 41.12 g (97.58 %).
(E)-2-Butenedioate (1/1) M.p.: 154-157.5 °C.

| Analysis for the formula $C_{25}H_{35}BrN_2O_6$ (537.45): | | | | |
|---|---|---|---|---|
| Calculated:<br>Found: | C % = 55.87<br>C % = 55.72 | H % = 6.19<br>H % = 6.13 | N % = 5.21<br>N % = 5.34 | Br % = 14.87<br>Br % = 14.83. |

UV: $\lambda_{max}$ = 274 nm ($\epsilon$ = 19069).

**Example 14**

**(R,S)-6-(E)-(2-Methoxyphenylmethylene)-2-methyl-1-(E)-[2-(dimethylamino)-ethoxyimino]-cyclohexane**

6-(E)-2-(2-Methoxyphenylmethylene)-2-methylcyclohexan-1-one-(E)-oxime (24.53 g; 0.1 mole) is converted into a salt in a saturated (40% by weight) aqueous solution of an alkali hydroxide (sodium and/or potassium hydroxide) in the presence of 20 $cm^3$ of dimethyl sulfoxide, and the salt thus obtained is reacted with 2-chloro-N,N-dimethylethylamine (11.83 g; 0.11 mole) at the boiling point of the reaction mixture. Further on the process of Example 1 is followed.
Yield: 26.86 g (84.88 %).
(E)-2-Butenedioate (1/1) M.p.: 152-157 °C.

| Analysis for the formula $C_{23}H_{32}N_2O_6$ (432.51): | | | |
|---|---|---|---|
| Calculated:<br>Found: | C % = 63.87<br>C % = 63.87 | H % = 7.46<br>H % = 7.55 | N % = 6.48<br>N % = 6.57. |

UV: $\lambda_{max}$ = 261 nm ($\epsilon$ = 12560).

**Example 15**

**(R,S)-6-(E)-(2-Methoxyphenylmethylene)-2-methyl-1-(E)-[3-(dimethylamino)-2-methylpropoxyimino)-cyclohexane**

One proceeds as specified in Example 14 except that instead of 2-chloro-N,N-dimethylethylamine 3-chloro-2,N,N-trimethylpropylamine (14.92 g; 0.11 mole) is used.
Yield: 24.51 g (71.15 %).
(E)-2-Butenedioate (1/1) M.p.: 143.5-147.5 °C.

| Analysis for the formula $C_{25}H_{36}N_2O_6$ (460.56): | | | |
|---|---|---|---|
| Calculated: | C % = 65.19 | H % = 7.88 | N % = 6.08 |
| Found: | C % = 65.10 | H % = 7.62 | N % = 6.18. |

UV: $\lambda_{max}$ = 265 nm ($\epsilon$ = 12871)
286 nm ($\epsilon$ = 9930).

**Example 16**

**(R,S)-6-(E)-(2,4-Dichlorophenylmethylene)-2-methyl-1-(E)-{3-[4-(phenylmethyl)-1-piperazinyl]-2-methylpropoxyimino}-cyclohexane**

One proceeds as specified in Example 1 except that instead of 6-(E)-phenylmethylene-2-methylcyclohexan-1-one-(E)-oxime 6-(E)-(2,4-dichlorophenylmethylene)-2-methyl-1-one-(E)-oxime (28.42 g; 0.1 mole) and instead of 3-chloro-N,N-dimethylpropylamine 1-(phenylmethyl)-4-(3-chloro-2-methyl-propyl)-piperazine (29.35 g; 0.11 mole) is used.
Yield: 20.56 g (39.96 %).
(E)-2-Butenedioate (1/2) M.p.: 205 to 210 °C.

| Analysis for the formula $C_{37}H_{45}Cl_2N_3O_9$ (746.66): | | | | |
|---|---|---|---|---|
| Calculated: | C % = 59.51 | H % = 6.07 | N % = 5.63 | Cl % = 9.50 |
| Found: | C % = 59.83 | H % = 6.21 | N % = 5.77 | Cl % = 9.35. |

UV: $\lambda_{max}$ = 248 nm ($\epsilon$ = 12161).

**Example 17**

**(R,S)-6-(E)-(2-Methoxy-4-ethoxyphenylmethylene)-2-methyl-1-(E)-{3-[4-(phenylmethyl)-1-piperazinyl]-propoxyimino}-cyclohexane**

One proceeds as specified in Example 1 except that instead of 6-(E)-phenylmethylene-2-methyl-1-one-(E)-oxime 6-(E)-(2-methoxy-4-ethoxyphenylmethylene)-2-methyl-1-one-(E)-oxime (28.94 g; 0.1 mole) and instead of 3-chloro-N,N-dimethylpropylamine 1-(phenylmethyl)-4-(3-chloropropyl)-piperazine (27.81 g; 0.11 mole) is used.
Yield: 34.25 g (67.73 %).
Hydrochloride (1/2) M.p.: 193-199 °C.

| Analysis for the formula $C_{31}H_{45}Cl_2N_3O_3$ (578.60): | | | | |
|---|---|---|---|---|
| Calculated: | C % = 64.35 | H % = 7.84 | N % = 7.26 | Cl % = 12.25 |
| Found: | C % = 64.34 | H % = 7.63 | N % = 7.19 | Cl % = 12.12. |

UV: $\lambda_{max}$ = 292 nm ($\epsilon$ = 16837).

**Example 18**

**(R,S)-6-(E)-Phenylmethylene-2-ethyl-1-(E)-[3-(dimethylamino)-2-methylpropoxyimino]-cyclohexane**

One proceeds according to Example 2 except that instead of benzene toluene is used and instead of 6-(E)-phenylmethylene-2-methylcyclohexan-1-(E)-oxime 6-(E)-phenylmethylene-2-ethyl-cyclohexan-1-one-(E)-oxime (22.93 g; 0.1 mole) is applied as starting substance.
Yield: 22.43 g (68.28 %).
(E)-2-Butenedioate (1/1) M.p.: 98 -101 °C.

| Analysis for the formula $C_{25}H_{36}N_2O_5$ (444.56): | | | |
|---|---|---|---|
| Calculated: | C % = 67.54 | H % = 8.16 | N % = 6.30 |
| Found: | C % = 67.58 | H % = 8.04 | N % = 6.30. |

UV: $\lambda_{max}$ = 268 nm ($\epsilon$ = 15620).

**Example 19**

**(R,S)-6-(E)-Phenylmethylene-2-ethyl-(E)-[3-(4-methyl-1-piperazinyl)-2-methylpropoxyimino]-cyclohexane**

One proceeds as specified in Example 18 except that instead of 3-chloro-2,N,N-trimethylpropylamine 1-(3-chloro-2-methylpropyl)-4-methylpiperazine (20.98 g; 0.11 mole) is used as starting substance.
Yield: 29.52 g (76.95 %).
(E)-2-Butenedioate (1/2) M.p.: 195-200 °C.

| Analysis for the formula $C_{32}H_{45}N_3O_9$ (615.7): | | | |
|---|---|---|---|
| Calculated: | C % = 62.42 | H % = 7.37 | N % = 6.82 |
| Found: | C % = 61.91 | H % = 6.93 | N % = 6.77. |

UV: $\lambda_{max}$ = 267 nm ($\epsilon$ = 15277).

**Example 20**

**(R,S)-6-(E)-(4-Methoxyphenylmethylene)-2-propyl-1-(E)-{2-[4-(phenylmethyl-1-piperazinyl]-ethoxyimino}-cyclohexane**

One proceeds as specified in Example 1, except that instead of benzene toluene is used, instead of 6-(E)-phenylmethylene-2-methylcyclohexan-1-one-(E)-oxime 6-(E)-(4-methoxyphenylmethylene)-2-propyl-1-one-(E)-oxime (27.34 g; 0.1 mole) and instead of 3-chloro-N,N-dimethylpropylamine 1-(phenylmethyl)-4-(2-chloroethyl)-piperazine (26.26 g; 0.11 mole) is applied.
Yield: 34.65 g (72.85 %).
(E)-2-Butenedioate (1/2) M.p.: 188-192 °C.

| Analysis for the formula $C_{38}H_{49}N_3O_{10}$ (707.79): | | | |
|---|---|---|---|
| Calculated: | C % = 64.48 | H % = 6.98 | N % = 5.94 |
| Found: | C % = 64.44 | H % = 7.00 | N % = 6.04. |

UV: $\lambda_{max}$ = 281 nm ($\epsilon$ = 19640).

**Example 21**

**(R,S)-6-(E)-(4-Methoxyphenylmethylene)-2-propyl-1-(E)-[2-(hexamethyleneimino)-ethoxyimino]-cyclohexane**

One proceeds as specified in Example 20 except that instead of 1-(phenylmethyl)-4-(2-chloroethyl)-piperazine N-(2-chloroethyl)-hexahydro-1H-azepine (17.78 g; 0.11 mole) is used.
Yield: 33.73 g (84.63 %).
(E)-2-Butenedioate (1/1) M.p.: 151-154 °C.

| Analysis for the formula $C_{29}H_{42}N_2O_6$ (514.65): | | | |
|---|---|---|---|
| Calculated: | C % = 67.68 | H % = 8.23 | N % = 5.44 |
| Found: | C % = 67.22 | H % = 8.40 | N % = 5.44. |

UV: $\lambda_{max}$ = 281 nm ($\epsilon$ = 20840).

**Example 22**

**(R,S)-6-(E)-Phenylmethylene-2-butyl-1-(E)-[3-(dimethylamino)-propoxyimino]-cyclohexane**

A solution of 6-(E)-phenylmethylene-2-butylcyclohexan-1-one (24.24 g; 0.1 mole) and O-[3-(dimethylamino)-propyl]-hydroxylamine (11.81 g; 0.1 mole) in ethanol is reacted at the boiling point of the mixture until the starting substance cannot be detected in it by thin layer chromatography (Kieselgel 60 $F_{245}$, benzene:methanol 4:1). Then fumaric acid (11.60 g; 0.1 mole) is added to the mixture and the separated crystals are filtered.
Yield: 26.55 g (77.5 %).
(E)-2-Butenedioate (1/1) M.p.: 112.7 to 115 °C.

| Analysis for the formula $C_{26}H_{38}N_2O_5$ (458.58): | | | |
|---|---|---|---|
| Calculated: | C % = 68.09 | H % = 8.35 | N % = 6.11 |
| Found: | C % = 68.10 | H % = 8.13 | N % = 5.88. |

UV: $\lambda_{max}$ = 270 nm.

**Example 23**

**(R,S)-6-(E)-Phenylmethylene-2-butyl-1-(E)-[3-(4-methyl-1-piperazinyl)-propoxyimino]-cyclohexane**

One proceeds as specified in Example 1 except that instead of 6-(E)-phenylmethylene-2-methylcyclohexan-1-one-(E)-oxime 6-(E)-phenylmethylene-2-butylcyclohexan-1-on-(E)-oxime (25.32 g; 0.1 mole) and instead of 3-chloro-N,N-dimethyl-propylamine 1-(3-chloropropyl)-4-methylpiperazine (19.44 g; 0.11 mole) is used.
Yield: 29.0 g (56.45 %).
(E)-2-Butenedioate (1/2) M.p.: 182 to 197.2 °C.

| Analysis for the formula $C_{33}H_{43}N_3O_9$ (629.76): | | | |
|---|---|---|---|
| Calculated: | C % = 62.96 | H % = 7.47 | N % = 6.67 |
| Found: | C % = 63.60 | H % = 7.61 | N % = 6.78. |

UV: $\lambda_{max}$ = 268 nm ($\epsilon$ = 16700).

**Example 24**

**(R,S)-6-(E)-Phenylmethylene-2-butyl-1-(E)-{2-[bis-(1-methylethyl)-amino]-ethoxyimino}-cyclohexane**

One proceeds as specified in Example 23 except that instead of 1-(3-chloropropyl)-4-methylpiperazine N-(2-chloroethyl)-N-(1-methylethyl)-2-propylamine (18.01 g; 0.11 mole) is used.
Yield: 34.50 g (89.70 %).
Hydrochloride (1/1) M.p.: 127 to 132 °C.

| Analysis for the formula $C_{25}H_{41}ClN_2O$ (421.06): | | | | |
|---|---|---|---|---|
| Calculated:<br>Found: | C % = 71.31<br>C % = 71.22 | H % = 9.82<br>H % = 9.97 | N % = 6.65<br>N % = 6.59 | Cl % = 8.42<br>Cl % = 8.26. |

UV: $\lambda_{max}$ = 273 nm ($\epsilon$ = 16215).

**Example 25**

**(R,S)-6-(E)-Phenylmethylene-2-butyl-1-(E)-[3-(4-phenylmethyl-1-piperazinyl)-propoxyimino]-cyclohexane**

One proceeds as specified in Example 23 except that instead of 1-(3-chloropropyl)-4-methylpiperazine 4-(phenylmethyl)-1-(3-chloropropyl)-piperazine (27.81 g; 0.11 mole) is used.
Yield: 43.21 g (91.22%).
Dihydrochloride (1/2) M.p.: 188 to 193 °C.

| Analysis for the formula $C_{31}H_{45}ClN_3O$ (546.64): | | | | |
|---|---|---|---|---|
| Calculated:<br>Found: | C % = 68.11<br>C % = 67.51 | H % = 8.30<br>H % = 8.59 | N % = 7.69<br>N % = 7.45 | Cl % = 12.97<br>Cl % = 12.36. |

UV: $\lambda_{max}$ = 273 nm ($\epsilon$ = 15830).

**Example 26**

**(R,S)-6-(E)-Phenylmethylene-2-butyl-1-(E)-[2-(dimethylamino)-ethoxyimino]-cyclohexane**

One proceeds as specified in Example 23 except that instead of 1-(3-chloropropyl)-4-methylpiperazine 2-chloro-N,N-dimethylethylamine (11.83 g; 0.11 mole) is used.
Yield: 15.71 g (49.64 %).
Hydrochloride (1/1) M.p.: 132 to 150 °C.

| Analysis for the formula $C_{20}H_{33}ClN_2O$ (352.94): | | | | |
|---|---|---|---|---|
| Calculated:<br>Found: | C % = 68.05<br>C % = 68.80 | H % = 9.42<br>H % = 9.25 | N % = 7.94<br>N % = 7.84 | Cl % = 10.05<br>Cl % = 9.66. |

UV: $\lambda_{max}$ = 273 nm ($\epsilon$ = 15180).

**Example 27**

**(R,S)-6-(E)-(4-Chlorophenylmethylene)-2-butyl-1-(E)-**

**[3-(dimethylamino)-2-methylpropoxyimino]-cyclohexane**

One proceeds as specified in Example 1 except that instead of 6-(E)-phenylmethylene-2-methylcyclohexan-1-one-(E)-oxime 6-(E)-(4-chlorophenylmethylene)-2-butylcyclohexan-1-one-(E)-oxime (29.19 g;

0.1 mole) and instead of 3-chloro-N,N-dimethylpropylamine 3-chloro-2,N,N-trimethylpropylamine (14.92 g; 0.11 mole is used.
Yield: 29.43 g (75.27 %).
(E)-2-Butenedioate (1/1) M.p.: 134 to 143 °C.

| Analysis for the formula $C_{27}H_{39}ClN_2O_5$ (507.063): | | | | |
|---|---|---|---|---|
| Calculated:<br>Found: | C % = 63.95<br>C % = 64.55 | H % = 7.75<br>H % = 7.84 | N % = 5.53<br>N % = 5.38 | Cl % = 6.99<br>Cl % = 7.03. |

UV: $\lambda_{max}$ = 272 nm ($\epsilon$ = 19388).

**Example 28**

**(R,S)-6-(E)-(4-Chlorophenylmethylene)-2-butyl-1-(E)-(2-piperidinyl-ethoxyimino)-cyclohexane**

One proceeds as specified in Example 27 except that instead of 3-chloro-2,N,N-trimethylpropylamine N-(2-chloroethyl)-piperidine (16.24 g; 0.11 mole) is used.
Yield: 25.36 g (62.93 %).
(E)-2-Butenedioate (1/1) M.p.: 160 to 165 °C.

| Analysis for the formula $C_{28}H_{39}ClN_2O_5$ (519.07): | | | | |
|---|---|---|---|---|
| Calculated:<br>Found: | C % = 64.75<br>C % = 64.52 | H % = 7.57<br>H % = 7.43 | N % = 5.40<br>N % = 5.39 | Cl % = 6.83<br>Cl % = 6.86. |

UV: $\lambda_{max}$ = 272 nm ($\epsilon$ = 19980).

**Example 29**

**(R,S)-6-(E)-(4-Chlorophenylmethylene)-2-butyl-1-(E)-(2-morpholinyl-ethoxyimino)-cyclohexane**

One proceeds as specified in Example 27 except that instead of 3-chloro-2,N,N-trimethylpropylamine 4-(2-chloroethyl)-morpholine (16.46 g; 0.11 mole) is used.
Yield: 38.49 g (95.04 %).
(E)-2-Butenedioate (1/1) M.p.: 145 to 148 °C.

| Analysis for the formula $C_{27}H_{37}ClN_2O_5$ (521.05): | | | | |
|---|---|---|---|---|
| Calculated:<br>Found: | C % = 62.23<br>C % = 62.94 | H % = 7.16<br>H % = 7.30 | N % = 5.38<br>N % = 5.57 | Cl % = 6.81<br>Cl % = 6.79. |

UV: $\lambda_{max}$ = 272 nm ($\epsilon$ = 18944).

**Example 30**

**(R,S)-6-(E)-(4-Fluorophenylmethylene)-2-pentyl-1-(E)-[2-(4-phenylmethyl-1-piperazinyl)-ethoxyimino]-cyclohexane**

One proceeds as specified in Example 1 except that instead of benzene toluene is used, instead of 6-(E)-phenyl-methylene-2-methylcyclohexan-1-one-(E)-oxime 6-(E)-(4-fluorophenylmethylene)-2-pentylcyclohexan-1-one-(E)-oxime (28.94 g; 0.1 mole) and instead of 3-chloro-N,N-dimethylpropylamine 1-(phenylmethyl)-4-(2-chloroethyl)-piperazine (26.26 g; 0.11 mole) is applied.
Yield: 39.17 g (79.67 %)
(E)-2-Butenedioate (1/2) M.p.: 189-193.5 °C.

| Analysis for the formula $C_{39}H_{50}FN_3O_9$ (723.81): | | | | |
|---|---|---|---|---|
| Calculated:<br>Found: | C % = 64.71<br>C % = 65.26 | H % = 6.96<br>H % = 7.18 | N % = 5.81<br>N % = 5.88 | F % = 2.63<br>F % = 2.50. |

UV: $\lambda_{max}$ = 268 nm ($\epsilon$ = 16080).

**Example 31**

**(R,S)-6-(E)-Phenylmethylene-2-hexyl-1-(E)-[3-(dimethylamino)-propoxyimino]-cyclohexane**

One proceeds as specified in Example 1 except that instead of benzene toluene is used and instead of 6-(E)-phenylmethylene-2-methylcyclohexan-1-one-(E)-oxime 6-(E)-phenylmethylene-2-hexylcyclohexan-1-one-(E)-oxime (28.54 g; 0.1 mole) is applied.
Yield: 27.3 g (73.70 %).
Hydrochloride (1/1) M.p.: 136 to 138 °C.

| Analysis for the formula $C_{24}H_{39}ClN_2O$ (407.03): | | | | |
|---|---|---|---|---|
| Calculated:<br>Found: | C % = 70.82<br>C % = 71.10 | H % = 9.66<br>H % = 9.88 | N % = 6.88<br>N % = 6.91 | Cl % = 8.71<br>Cl % = 8.68. |

UV: $\lambda_{max}$ = 274 nm ($\epsilon$ = 17789).

**Example 32**

**(R,S)-6-(E)-(2-Methoxyphenylmethylene)-2-hexyl-1-(E)-(2-pyrrolidinylethoxyimino)-cyclohexane**

One proceeds as specified in Example 1 except that instead of benzene toluene is used, instead of 6-(E)-phenylmethylene-2-methylcyclohexan-1-one-(E)-oxime 6-(E)-(2-methoxyphenylmethylene)-2-hexylcyclohexan-1-one-(E)-oxime (31.54 g; 0.1 mole) and instead of 3-chloro-N,N-dimethylpropylamine 1-(2-chloroethyl)-pyrrolidine (14.70 g; 0.11 mole) is applied.
Yield: 29.49 g (71.47 %).
(E)-2-Butenedioate (1/1) M.p.: 142.5-147 °C.

| Analysis for the formula $C_{30}H_{44}N_2O_6$ (528.67): | | | |
|---|---|---|---|
| Calculated:<br>Found: | C % = 68.15<br>C % = 67.95 | H % = 8.39<br>H % = 8.51 | N % = 5.30<br>N % = 5.47. |

UV: $\lambda_{max}$ = 263 nm ($\epsilon$ = 12992).

**Example 33**

**(R,S)-6-(E)-(2-Methoxyphenylmethylene)-2-hexyl-1-(E)-[2-(diethylamino)-ethoxyimino]-cyclohexane**

One proceeds as specified in Example 32 except that instead of 1-(2-chloroethyl)-pyrrolidine 2-chloro-N,N-diethylethylamine (14.92 g; 0.11 mole) is used.
Yield: 18.98 g (45.78 %).
Hydrochloride (1/1) M.p.: 155.5-158.5 °C.

| Analysis for the formula $C_{26}H_{43}ClN_2O_2$ (451.08): | | | | |
|---|---|---|---|---|
| Calculated:<br>Found: | C % = 69.23<br>C % = 69.62 | H % = 9.61<br>H % = 9.33 | N % = 6.21<br>N % = 6.42 | Cl % = 7.86<br>Cl % = 7.84. |

UV: $\lambda_{max}$ = 263 nm ($\epsilon$ = 12204)
$\lambda_{max}$ = 294 nm ($\epsilon$ = 8844).

**Example 34**

**(R,S)-6-(E)-(4-Fluorophenylmethylene)-2-heptyl-1-(E)-[2-(dimethylamino)-ethoxyimino]-cyclohexane**

One proceeds as specified in Example 1 except that instead of 6-(E)-phenylmethylene-2-methylyclohexan-1-(E)-oxime 6-(E)-(4-fluorophenylmethylene)-2-heptylcyclohexan-1-(E)-one oxime (31.74 g; 0.1 mole) and instead of 3-chloro-N,N-dimethylpropylamine 2-chloro-N,N-dimethylethylamine (11.83 g; 0.11 mole) is used.
Yield: 31.3 g (80.55 %).
(E)-2-Butenedioate (1/1) M.p.: 92-99 °C.

| Analysis for the formula $C_{28}H_{41}FN_2O_5$ (504.63): | | | | |
|---|---|---|---|---|
| Calculated:<br>Found: | C % = 66.64<br>C % = 66.82 | H % = 8.19<br>H % = 8.29 | N % = 5.55<br>N % = 5.61 | R % = 3.77<br>R % = 3.70. |

UV: $\lambda_{max}$ = 265 nm ($\epsilon$ = 15593).

**Example 35**

**(R,S)-5-(E)-(2-Methoxyphenylmethylene)-2-butyl-1-(E)-[2-(diethylamino)-ethoxyimino]-cyclopentane**

5-(E)-(2-Methoxyphenylmethylene)-cyclopentan-1-one-(E)-oxime (27.24 g; 0.1 mole) is transformed into a salt with sodium amide (3.9 g; 0.1 mole, 50% by weight toluene suspension) in a mixture of dimethylformamide and toluene, in a ratio by volume of 1 : 1,
and the salt thus obtained is reacted with 2-chloro-N,N-diethylethylamine (14.92 g; 0.11 mole) at the boiling point of the reaction mixture. Further on the process of Example 1 is followed.
Yield: 30.96 g (83.10 %).
(E)-2-Butenedioate (1/1) M.p.: 134-138 °C.

| Analysis for the formula $C_{29}H_{44}N_2O_6$ (516.66): | | | |
|---|---|---|---|
| Calculated:<br>Found: | C % = 67.42<br>C % = 67.75 | H % = 8.58<br>H % = 8.68 | N % = 5.42<br>N % = 5.57. |

UV: $\lambda_{max}$ = 298 nm ($\epsilon$ = 15105)
$\lambda_{max}$ = 319 nm ($\epsilon$ = 15717).

**Example 36**

**(R,S)-5-(E)-(2-Methoxyphenylmethylene)-2-butyl-1-(E)-{2-[bis-(1-methylethyl)-amino]-ethoxyimino}-cyclopentane**

One proceeds as specified in Example 35 except that instead of 2-chloro-N,N-diethylethylamine N-(2-chloroethyl)-N-(1-methylethyl)-2-propylamine (18.01 g; 0.11 mole) is used.
Yield: 30.96 g (83.11 %).
(E)-2-Butenedioate (1/1) M.p.: 134-138 °C.

| Analysis for the formula $C_{29}H_{44}N_2O_6$ (516.66): | | | |
|---|---|---|---|
| Calculated:<br>Found: | C % = 67.42<br>C % = 67.75 | H % = 8.58<br>H % = 8.68 | N % = 5.42<br>N % = 5.57. |

UV: $\lambda_{max}$ = 298 nm ($\epsilon$ = 15105)
$\lambda_{max}$ = 319 nm ($\epsilon$ = 15717).

**Example 37**

**(R,S)-5-(E)-(4-Chlorophenylmethylene)-2-butyl-1-(E)-[3-(dimethylamino)-propoxyimino]-cyclopentane**

One proceeds as specified in Example 1 except that instead of benzene toluene is used and instead of 6-(E)-phenyl-methylenecyclohexan-1-one-(E)-oxime 5-(E)-(4-chlorophenylmethylene)-cyclopentan-1-one-(E)-oxime (27.78 g; 0.1 mole) is applied.
Yield: 27.33 g (75.31 %).
(E)-2-Butenedioate (1/1) M.p.: 131-134 °C.

| Analysis for the formula $C_{25}H_{35}ClN_2O_5$ (479.007): | | | | |
|---|---|---|---|---|
| Calculated:<br>Found: | C % = 62.68<br>C % = 62.32 | H % = 7.37<br>H % = 7.40 | N % = 5.85<br>N % = 5.96 | Cl % = 7.40<br>Cl % = 7.45. |

UV: $\lambda_{max}$ = 305 nm ($\epsilon$ = 32931).

**Example 38**

**(R,S)-7-(E)-(4-Chlorophenylmethylene)-2-propyl-1-(E)-[2-(diethylamino)-ethoxyimino]-cycloheptane**

7-(E)-(4-Chlorophenylmethylene)-2-propylcycycloheptan-1-(E)-oxime (27.98 g; 0.1 mole) is transformed into a salt with sodium hydride (4.8 g; 0.1 mole, 50% by weight oily dispersion) in a mixture of 30 $cm^3$ of dimethylformamide and 200 $cm^3$ of benzene, and the salt thus obtained is reacted with 2-chloro-N,N-diethylethylamine (14.92 g; 0.11 mole). Further on the process of Example 1 is followed.
Yield: 32.26 g (82.5 %).
(E)-2-Butenedioate (1/1) M.p.: 96-99 °C.

| Analysis for the formula $C_{27}H_{39}ClN_2O_5$ (507.06): | | | | |
|---|---|---|---|---|
| Calculated:<br>Found: | C % = 63.95<br>C % = 63.3 | H % = 7.75<br>H % = 7.91 | N % = 5.53<br>N % = 5.53 | Cl % = 6.99<br>Cl % = 6.83. |

UV: $\lambda_{max}$ = 240 nm ($\epsilon$ = 18718).

**Example 39**

**(R,S)-6-(E)-(3,4-Dichlorophenylmethylene)-2-methyl-1-(E)-[2-(diethylamino)-ethoxyimino]-cyclohexane**

One proceeds as specified in Example 38 except that instead of 7-(E)-(4-chlorophenylmethylene)-2-propylcycloheptan-1-(E)-oxime 6-(E)-(3,4-dichlorophenylmethylene)-2-methylcyclohexan-1-one-(E)-oxime (29.3 g; 0.1 mole) is used.
Yield: 30.4 g (79.3 %).
(E)-2-Butenedioate (1/1) M.p.: 126-129 °C.

| Analysis for the formula $C_{24}H_{32}Cl_2N_2O_5$ (499.43): | | | | |
|---|---|---|---|---|
| Calculated:<br>Found: | C % = 57.71<br>C % = 57.13 | H % = 6.46<br>H % = 6.30 | N % = 5.61<br>N % = 5.73 | Cl % = 14.2<br>Cl % = 14.3. |

UV: $\lambda_{max}$ = 274 nm ($\epsilon$ = 10348).

**Example 40**

**(R,S)-6-(E)-(4-Chlorophenylmethylene)-2-methyl-1-(E)-{2-[bis(1-methylethyl)-amino]-ethoxyimino}-cyclohexane**

One proceeds as specified in Example 24 except that instead of 6-(E)-phenylmethylene-2-methylcyclohexan-1-one-(E)-oxime 6-(E)-(4-chlorophenylmethylene)-2-methylcyclohexan-1-one-(E)-oxime (25.57 g; 0.1 mole) is used.
Yield: 29.3 g (77.7 %).
(E)-2-Butenedioate (1/1) M.p.: 127-129 °C.

| Analysis for the formula $C_{26}H_{37}ClN_2O_5$ (493.04): | | | | |
|---|---|---|---|---|
| Calculated: | C % = 63.33 | H % = 7.57 | N % = 5.68 | Cl % = 7.19 |
| Found: | C % = 63.27 | H % = 7.68 | N % = 5.71 | Cl % = 7.06. |

UV: $\lambda_{max}$ = 272 nm ($\epsilon$ = 19197).

**Example 41**

**(R,S)-6-(E)-(4-Chlorophenylmethylene)-2-methyl-1-(E)-[3-(dimethylamino)-2-methylpropoxyimino]-cyclohexane**

One proceeds as specified in Example 9 except that instead of 6-(E)-(4-fluorophenylmethylene)-2-methylcyclohexan-1-one-(E)-oxime 6-(E)-(4-chlorophenylmethylene)-2-methylcyclohexan-1-one-(E)-oxime (23.57 g; 0.1 mole) is used.
Yield: 22.16 g (63.5 %)
(E)-2-Butenedioate (1/1) M.p.: 186-189 °C.

| Analysis for the formula $C_{24}H_{33}ClN_2O_5$ (464.98): | | | | |
|---|---|---|---|---|
| Calculated: | C % = 61.99 | H % = 7.15 | N % = 6.03 | Cl % = 7.63 |
| Found: | C % = 61.52 | H % = 7.01 | N % = 5.93 | Cl % = 7.54. |

UV: $\lambda_{max}$ = 276 nm ($\epsilon$ = 19242).

**Example 42**

**(R,S)-2-Propyl-7-(E)-(4-chlorophenylmethylene)-1-(E)-[2-hydroxy-3-(N-pyrrolidinyl)propoxyimino]-cycloheptane**

a) One proceeds as specified in Example 7 except that 29.1 g (0.1 mole) of 2-propyl-7-(E)-(4-chlorophenylmethylene)cycloheptan-1-one-(E)-oxime is used as oxime. Yield: 34.3 g (99.5 %)

b) To a solution of the product obtained as specified above 14.22 g (0.2 mole) of pyrrolidine are added and the reaction mixture is boiled for 8 hours.

Yield: 32.85 g (78 %).
2-(E)-Butenedioate (1/1) M.p.: 166-168.5 °C.

| Analysis for the formula $C_{28}H_{33}ClN_2O_6$ (535.09): | | | | |
|---|---|---|---|---|
| Calculated: | C % = 62.85 | H % = 7.35 | N % = 5.24 | Cl % = 6.62 |
| Found: | C % = 62.51 | H % = 7.45 | N % = 5.19 | Cl % = 6.66. |

UV: $\lambda_{max}$ = 261 nm ($\epsilon$ = 19471).

**Example 43**

Tablet comprising 25 mg of active ingredient

a) The composition of one tablet is as follows:

| | |
|---|---|
| active ingredient | 25.0 mg |
| corn starch | 97.0 mg |
| polyvinyl-pyrrolidone | 175.0 mg |
| magnesium stearate | 3.0 mg |
| | 300.0 mg |

The tablet is prepared as follows:

The active ingredient and the corn starch are admixed, then wetted with 10 to 15 % by weight of aqueous polyvinyl-pyrrolidone solution and the mixture is granulated, then dried at a temperature of 40 to 50 °C. The dry granules are rubbed through a sieve, mixed with magnesium stearate and tablets are prepared from the mixture.

The weight of one tablet is 300.0 mg.

b) The composition of one tablet is as follows:

| | |
|---|---|
| active ingredient | 25.0 mg |
| magnesium stearate | 0.5 mg |
| stearine | 0.5 mg |
| talc | 1.0 mg |
| gelatin | 1.7 mg |
| microcrystalline cellulose | 6.0 mg |
| corn starch | 15.3 mg |
| lactose | 50.0 mg |
| | 100.0 mg |

The tablet is prepared as follows:

The active ingredient, the corn starch, th lactose and the cellulose are admixed and granulated with 10% by weight gelatin solution then dried at a temperature of 40 to 50°C. The dry granules are rubbed through a sieve, mixed with talc, stearine and magnesium stearate and tablets are prepared from the mixture.

The weight of one tablet is 100.0 mg.

**Example 44**

Tablet comprising 250 mg of active ingredient

The composition of one tablet is as follows:

| | |
|---|---|
| active ingredient | 250.0 mg |
| lactose | 270.0 mg |
| corn starch | 75.0 mg |
| magnesium stearate | 5.0 mg |
| | 600.0 mg |

The active ingredient, the lactose and the corn starch are wetted and mixed, granulated and dried at a temperature of 40 to 50 °C. The dry granules are rubbed through a sieve as described hereinabove, mixed with magnesium stearate, then tablets are formed.

The weight of one tablet is 600.0 mg.

**Example 45**

Dragée comprising 25 mg of active ingredient

The composition of one dragée core is as follows:

| | |
|---|---|
| active ingredient | 25.0 mg |
| corn starch | 245.0 mg |
| talc | 18.0 mg |
| gelatin | 8.0 mg |
| magnesium stearate | 4.0 mg |
| | 300.0 mg |

The active ingredient and the corn starch are mixed, wetted with 10 % by weight aqueous gelatin solution, granules are formed from the wet mixture, then the granules are dried at a temperature of 40 to 50 °C. The dry granules are rubbed through a sieve, homogenized with talc and magnesium stearate and dragée cores of 300.0 mg are compressed from the mixture.

**Example 46**

Dragée comprising 50.0 mg of active ingredient

The composition of one dragée core is as follows:

| | |
|---|---|
| active ingredient | 50.0 mg |
| lactose | 97.0 mg |
| polyvinyl-pyrrolidone | 2.0 mg |
| magnesium stearate | 1.0 mg |
| | 150.0 mg |

The granules are prepared as described hereinabove. The weight of the dragée cores is 150 mg.

The dragée cores are coated with a layer containing sugar and talc in a manner known per se. The dragée thus obtained is painted with non-toxic food paint to the desired colour and polished with bees-wax.

**Example 47**

Gelatin capsule comprising 5.0 mg of active ingredient

The composition of one gelatin capsule is as follows:

| | |
|---|---|
| active ingredient | 5.0 mg |
| corn starch | 40.0 mg |
| Aerosil® | 3.0 mg |
| magnesium stearate | 2.0 mg |
| | 50.0 mg |

The ingredients are homogenized and filled into gelatin capsules of suitable size.

**Example 48**

Gelatin capsule comprising 25.0 mg of active ingredient

The composition of one gelatin capsule is as follows:

| | |
|---|---|
| active ingredient | 25.0 mg |
| corn starch | 265.0 mg |
| Aerosil® | 6.0 mg |
| magnesium stearate | 4.0 mg |
| | 300.0 mg |

The ingredients are homogenized and filled into gelatin capsules of suitable size.

**Example 49**

Gelatin capsule comprising 50.0 mg of active ingredient

The composition of one gelatin capsule is as follows:

| | |
|---|---|
| active ingredient | 50.0 mg |
| lactose | 90.0 mg |
| Aerosil® | 6.0 mg |
| magnesium stearate | 4.0 mg |
| | 150.0 mg |

The ingredients are homogenized and filled into gelatin capsules of suitable size.

**Example 50**

Gelatin capsule comprising 250.0 mg of active ingredient

The composition of one gelatin capsule is as follows:

| | |
|---|---|
| active ingredient | 250.0 mg |
| lactose | 148.0 mg |
| magnesium stearate | 2.0 mg |
| | 400.0 mg |

The ingredients are homogenized and filled into gelatin capsules of suitable size.

**Example 51**

Injection solution comprising 25.0 mg of active ingredient

The composition of one ampoule is as follows:

| | |
|---|---|
| active ingredient | 25.0 mg |
| sodium chloride | 5.0 mg |
| dissolved in 5 $cm^3$ of twice-distilled water. | |

The active ingredient and the sodium chloride are dissolved in the necessary amount of twice-distilled water suitable for making injections. The solution is filtered, filled into ampoules and sterilized.

**Example 52**

Injection solution comprising 50.0 mg of active ingredient

The composition of one ampoule is as follows:

| | |
|---|---|
| active ingredient | 50.0 mg |
| sodium chloride | 10.0 mg |

The active ingredient and the sodium chloride are dissolved in the necessary amount of twice-distilled water, then filled into ampoules under sterile conditions.

Example 53

Suppository comprising 250 mg of active ingredient

The composition of one suppository is as follows:

| | |
|---|---|
| active ingredient | 250.0 mg |
| fatty acid glyceride | 750.0 mg |

The fatty acid glyceride is melted, the active ingredient is homogenized, then poured into a mould. One suppository weights 1000.0 mg and comprises 250.0 mg of active ingredient.

**Example 54**

Drop comprising 5 % by weight of active ingredient

The composition of the drop-solution is as follows:

| | |
|---|---|
| active ingredient | 50.0 mg |
| sorbitol | 340.0 mg |
| polyethylene glycol | 100.0 mg |
| citric acid | 1.0 mg |
| sodium citrate | 3.0 mg |
| ion-free water | 505.0 mg |
| flavourant | 1.0 mg |
| | 1 000.0 mg |

The sorbitol, the active ingredient, citric acid and sodium citrate are dissolved in the aqueous solution of polyethylene glycol, then after dissolution of the solid materials the flavourant is added. The solution is filtered and filled into flasks supplied with a drop-dispenser.

**Claims**

**1.** (Phenylmethylene)-2-(alkyl)-1-[ω-(amino)-alkoxyimino]-cycloalkane derivatives of general formula

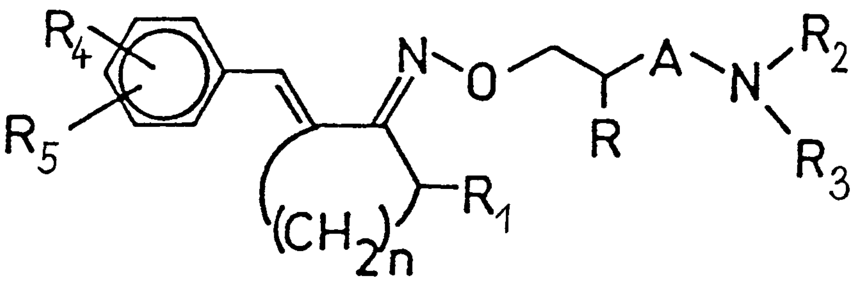

I ,

wherein

| | |
|---|---|
| R | represents hydrogen, $C_{1-4}$ alkyl or hydroxyl, |
| $R_1$ | stands for $C_{1-12}$ alkyl, |
| $R_2$ and $R_3$ | independently each represents hydrogen, a $C_{1-12}$ alkyl group, optionally substituted by hydroxy, or a $C_{2-12}$ alkenyl group |
| | or |
| $R_2$ and $R_3$ | together with the nitrogen atom to which they are attached form a 4- to 7-membered heterocyclic ring, optionally comprising an oxygen, sulfur or a further nitrogen atom, which latter optionally may carry a phenyl, benzyl or $C_{1-4}$ alkyl substituent, |
| $R_4$ and $R_5$ | independently each stands for hydrogen, halogen or a $C_{1-4}$ alkoxy group or together represent a 3,4-methylenedioxy group, |
| n | is an integer from 2 to 5 |
| | and |
| A | represents a valency bond or a $-CH_2$-group, |

as well as stereo and optically active isomers and their mixtures, acid-addition salts and quaternary ammonium derivatives thereof.

**2.** Cycloalkane derivatives according to claim 1, characterized in that the $C_{1-4}$ alkyl group which may be represented by R and/or the $C_{1-4}$ alkyl substituent by which the optional further nitrogen atom of the 4- to 7-membered heterocyclic ring which may be formed by $R_2$ and $R_3$ together with the nitrogen atom to which they are attached may be substituted and/or the $C_{1-4}$ alkoxy group(s) which may be represented by $R_4$ and/or $R_5$ is/are such having from 1 to 3, particularly 1 or 2, carbon atom(s) and/or the $C_{1-12}$ alkyl group(s) which may be represented by $R_2$ and/or $R_3$ is/are such having from 1 to 8, particularly 1 to 4, carbon atom(s) and/or the $C_{1-12}$ alkyl group which may be represented by $R_1$ is such having from 1 to 8, particularly 1 to 7, carbon atom(s) and/or the $C_{2-12}$ alkenyl group(s) which may be represented by $R_2$ and/or $R_3$ is/are such having from 2 to 6, particularly 2 to 4, carbon atom(s) and/or the 4- to 7-membered heterocyclic ring which may be formed by $R_2$ and $R_3$ together with the nitrogen atom to which they are attached is such having from 5 to 7, particularly 5 or 6, carbon atoms, most particularly a morpholino, pyrrolidinyl, piperidinyl, piperazinyl or hexamethyleneimino ring, and/or the halogen atom(s) which may be represented by $R_4$ and/or $R_5$ is/are chlorine, fluorine and/or bromine and/or the integer represented by n is from 2 to 4, particularly 3 or 4.

**3.** Cycloalkane derivatives according to claim 1 or 2, characterized in that

| | |
|---|---|
| $R_1$ | stands for $C_{1-8}$ alkyl, |
| $R_2$ and $R_3$ | independently each represents $C_{1-4}$ alkyl |
| | or |
| $R_2$ and $R_3$ | together with the nitrogen atom to which they are attached form a 5- or 6-membered heterocyclic ring, optionally comprising a further nitrogen atom, which latter optionally may carry a benzyl substituent, |
| $R_4$ and $R_5$ | independently each stands for hydrogen, halogen or methoxy, |
| n | is 3 or 4 |
| | and |
| A and R | are as defined in claim 1 or 2. |

**4.** (R,S)-6-(E)-[4'-(Fluoro)-phenylmethylene]-2-[pentyl]-1-(E)-[2''-{4'''-(phenylmethyl)-piperazin-1'''-yl}-ethoxyimino]-cyclohexane,

(R,S)-2-[propyl]-7-(E)-[4'-(chloro)-phenylmethylene]-1-(E)-[2''-{hydroxy}-3''-(N-pyrrolidinyl)-propoxyimino]-cycloheptane,
(R,S)-6-(E)-[4'-(fluoro)-phenylmethylene]-2-[methyl]-1-(E)-[3''-(dimethylamino)-2''-(methyl)-propoxyimino]-cyclohexane,
(R,S)-6-(E)-[2'-(methoxy)-phenylmethylene]-2-[hexyl]-1-(E)-[2''-(diethylamino)-ethoxyimino]-cyclohexane,
(R,S)-6-(E)-[4'-(fluoro)-phenylmethylene]-2-[heptyl]-1-(E)-[2''-(dimethylamino)-ethoxyimino]-cyclohexane,
(R,S)-7-(E)-[4'-(chloro)-phenylmethylene]-2-[propyl]-1-(E)-[2''-(diethylamino)-ethoxyimino]-cycloheptane and
(R,S)-6-(E)-[3',4'-di-(chloro)-phenylmethylene]-2-[methyl]-1-(E)-[2''-(diethylamino)-ethoxyimino]-cyclohexane
as well as stereo and optically active isomers and their mixtures, acid-addition salts and quaternary ammonium derivatives thereof.

**5.** A process for preparing the compounds according to claims 1 to 4, characterized in that in a manner known per se

a) (phenylmethylene)-2-(alkyl)-cycloalkane-1-one or -1-thione derivatives of general formula

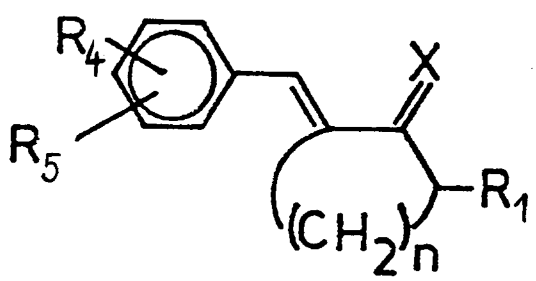

II ,

wherein $R_1$, $R_4$, $R_5$ and n are as defined in claims 1 to 3 and X stands for oxygen or sulfur, are reacted with substituted alkyl-0-hydroxylamines of general formula

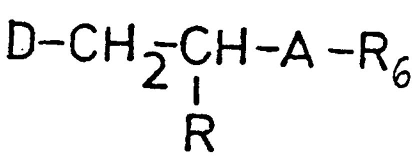

III,

wherein D represents a group of formula $H_2N$-0-, $R_6$ stands for a group of formula

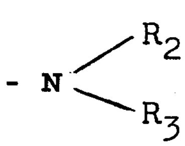

,

in which latter $R_2$ and $R_3$ are as defined in claims 1 to 3, and R and A are as defined in claims 1 to 3, or in the presence of a basic condensing agent with acid-addition salts thereof

or

b) for preparing compounds of general formula I, wherein R represents hydrogen or $C_{1-4}$ alkyl and $R_1$ , $R_2$ , $R_3$ , $R_4$ , $R_5$ , A and n are as defined in claims 1 to 3, (phenylmethylene)-2-(alkyl)-cycloalkane-1-oxime derivatives of general formula

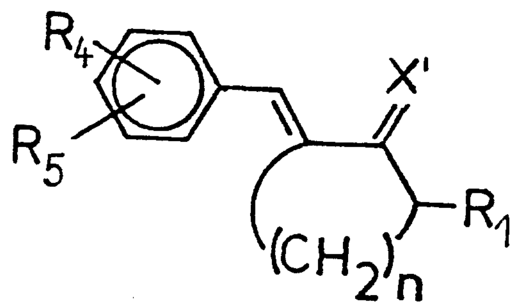

II' ,

wherein $R_1$, $R_4$, $R_5$ and n are as defined in claims 1 to 3 and X' stands for a group of formula = N - OH, are reacted with substituted alkylhalides of general formula

$$D' - CH_2 - \underset{\displaystyle R}{\underset{|}{CH}} - A - R_6 \qquad III' ,$$

wherein D' stands for halogen, R represents hydrogen or $C_{1-4}$ alkyl, $R_6$ is a group of formula

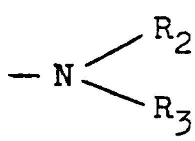

,

in which latter $R_2$ and $R_3$ are as defined in claims 1 to 3, and A is as defined in claim 1, or with acid-addition salts thereof in the presence of a basic condensing agent

or

c) for preparing compounds of general formula I, wherein R represents hydroxy, A stands for a group of formula - $CH_2$ - and $R_1$ , $R_2$ , $R_3$ , $R_4$, $R_5$ and n are as defined in claims 1 to 3, (phenylmethylene)-2-(alkyl)-cycloalkane-1-oxime derivatives of general formula II', wherein X' represents a group of formula = N - 0H and $R_1$, $R_4$, $R_5$ and n are as defined in claims 1 to 3, are reacted with 2,3-(epoxy)-propoxyhalides of general formula

$$D' - CH_2 - \underset{\displaystyle R''}{\underset{|}{CH}} - A'' - R_6'' \qquad III'' ,$$

wherein D' represents halogen and A'', $R_6$'' and R'' together form a group of formula $-CH_2-0-$, in the presence of a basic condensing agent, and the thus obtained (phenylmethylene)-2-(alkyl)-1-[2',3'-(epoxy)-propoxyimino]-cycloalkanes of general formula

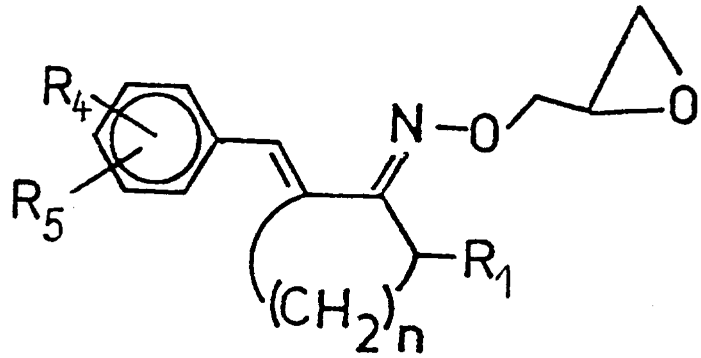

V,

wherein $R_1$, $R_4$, $R_5$ and n are as defined in claims 1 to 3, are reacted with amines of general formula

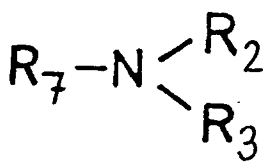

IV ,

wherein $R_7$ stands for hydrogen, and $R_2$ and $R_3$ are as defined in claims 1 to 3, or in the presence of a basic agent with acid-addition salts of the former or

d) for preparing compounds of general formula I, wherein R represents hydrogen or $C_{1-4}$ alkyl and $R_1$, $R_2$, $R_3$, $R_4$, $R_5$, A and n are as defined in claims 1 to 3 (phenylmethylene)-2-(alkyl)-cycloalkane-1-oxime derivatives of general formula II', wherein X' represents a group of formula = N - 0H and $R_1$, $R_4$, $R_5$ and n are as defined in claims 1 to 3, are reacted with $\alpha,\omega$-dihalogenalkanes of general formula

$$D' - CH_2 - \underset{\displaystyle R}{\underset{|}{CH}} - A - R_6''' \qquad III''',$$

wherein D' stands for halogen, R represents hydrogen or $C_{1-4}$ alkyl, $R_6'''$ means halogen and A is as defined in claim 1, in the presence of a basic condensing agent and the thus obtained (phenylmethylene)-2-(alkyl)-1-[$\omega$-(halogen)-alkoxyimino]-cycloalkane derivatives of general formula

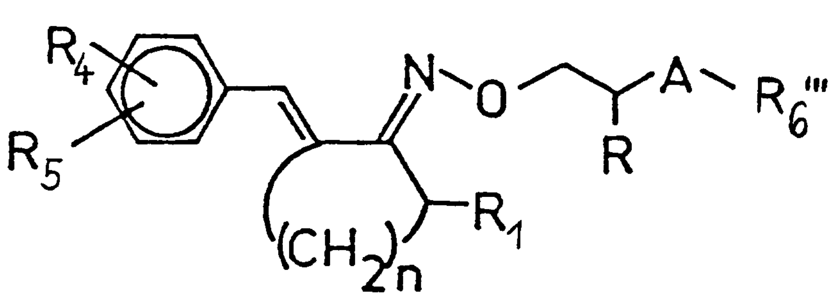

V' ,

wherein $R_1$, $R_4$, $R_5$, A and n are as defined in claims 1 to 3 and $R_6'''$ is as defined for formula III''', are reacted with amines of general formula

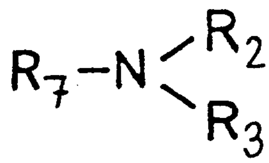

IV,

wherein $R_7$ stands for hydrogen, and $R_2$ and $R_3$ are as defined in claims 1 to 3, or in the presence of a basic agent with acid-addition salts of the former,

and optionally in a manner known per se (phenylmethylene)-2-(alkyl)-1-[ω-(amino)-alkoxyimino]-cycloalkane derivatives of general formula I thus obtained are converted into acid-addition salts or quaternary ammonium derivatives thereof or salts of (phenylmethylene)-2-(alkyl)-1-[ω-(amino)-alkoxyimino]-cycloalkane derivatives of general formula I thus obtained are converted into the free (phenylmethylene)-2-(alkyl)-1-[ω-(amino)-alkoxyimino]-cycloalkane derivative bases and/or into other acid-addition salts and/or mixtures or racemates of the above compounds of formula I are separated to the stereo and/or optically active isomers of the above compounds of formula I or the optically active isomers of the above compounds of formula I are racemised.

**6.** A process according to claim 5 a), characterized in that the reaction is carried out in inert solvents or in mixtures of such solvents, particularly in an aliphatic alcohol, pyridine or triethylamine or in a mixture thereof.

**7.** A process according to claim 5 b), characterized in that the reaction is carried out in inert solvents or mixtures of such solvents, particularly in an aliphatic or cyclic ether, aromatic hydrocarbon, dimethylformamide, dimethyl acetamide or dimethyl sulfoxide or in a mixture thereof.

**8.** A process according to claim 5 c) or d), characterized in that the reaction of the (phenylmethylene)-2-(alkyl)-cycloalkane-1-oxime derivatives of general formula II' with the 2,3-(epoxy)-propoxyhalides of formula III'' or α,ω-dihalogenalkanes of general formula III''' respectively, is carried out in inert or relatively inert solvents, particularly water, an aliphatic alcohol, aromatic hydrocarbon or aliphatic or cyclic ether or in a mixture thereof.

**9.** Medicaments, characterized in that they contain as [an] active ingredient(s) at least one compound according to claims 1 to 4, suitably in admixture with at least one inert solid and/or liquid pharmaceutical carrier and/or diluent and/or excipient.

**10.** Use of the compounds of claims 1 to 4 for preparing medicaments, particularly having ulcus and gastric-acid-secretion inhibiting effects.